(19) **Europäisches Patentamt**
**European Patent Office**
**Office européen des brevets**

(11) **EP 2 539 463 B1**

(12) **EUROPEAN PATENT SPECIFICATION**

(45) Date of publication and mention
of the grant of the patent:
**26.07.2017 Bulletin 2017/30**

(51) Int Cl.:
***C12Q 1/68*** (2006.01)

(21) Application number: **11747923.8**

(22) Date of filing: **22.02.2011**

(86) International application number:
**PCT/US2011/025687**

(87) International publication number:
**WO 2011/106310 (01.09.2011 Gazette 2011/35)**

(54) **MARKERS FOR OBESITY AND METHODS OF USE THEREOF**

MARKER FÜR ADIPOSITAS UND VERFAHREN ZU IHRER VERWENDUNG

MARQUEURS DE L'OBÉSITÉ ET LEURS PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.10.2010 US 392679 P**
**23.02.2010 US 307205 P**

(43) Date of publication of application:
**02.01.2013 Bulletin 2013/01**

(73) Proprietor: **The Children's Hospital of Philadelphia
Philadelphia, PA 19104 (US)**

(72) Inventors:
• **GRANT, Struan**
**Swarthmore, PA 19081 (US)**
• **GLESSNER, Joseph**
**Turnersville, NJ 08012 (US)**
• **HAKONARSON, Hakon**
**Malvern, PA 19355 (US)**

(74) Representative: **Potter Clarkson LLP
The Belgrave Centre
Talbot Street
Nottingham NG1 5GG (GB)**

(56) References cited:
• **DATABASE GeO [Online] 5 February 2008
(2008-02-05), "Platform GPL6433", XP002699053,
retrieved from NCBI Database accession no.
GPL6433**
• **DATABASE GeO [Online] 5 February 2008
(2008-02-05), "Platform GPL6434", XP002699054,
retrieved from NCBI Database accession no.
GPL6434**

• **WALTERS ROBIN G ET AL: "Rare Genomic
Structural Variants in Complex Disease: Lessons
from the Replication of Associations with
Obesity", PLOS ONE, vol. 8, no. 3, E58048, March
2013 (2013-03), pages 1-11, XP055067189, ISSN:
1932-6203**
• **GUNDERSON K L ET AL: "A genome-wide
scalable SNP genotyping assay using microarray
technology", NATURE GENETICS, vol. 37, no. 5,
17 April 2005 (2005-04-17), pages 549-554,
XP002396335, NATURE PUBLISHING GROUP,
NEW YORK, US ISSN: 1061-4036, DOI:
10.1038/NG1547**
• **RICHARDS ET AL.: 'A Genome-Wide Association
Study Reveals Variants in ARL15 that Influence
Adiponectin Levels. Art.nr e1000768' PLOS
GENET vol. 5, no. 12, December 2009, pages 1 -
10, XP008157622**
• **BOCHUKOVA ET AL.: 'Large, rare chromosomal
deletions associated with severe early-onset
obesity.' NATURE vol. 463, no. 7281, 06 December
2009, pages 666 - 670, XP008157930**
• **WILLER ET AL.: 'Six new loci associated with
body mass index highlight a neuronal influence
on body weight regulation.' NAT GEN vol. 41, no.
1, January 2009, pages 25 - 34, XP008157628**
• **WALTERS ET AL.: 'A new highly penetrant form
of obesity due to deletions on chromosome
16p11.2.' NATURE vol. 463, no. 7281, 04 February
2010, pages 671 - 674, XP008157929**
• **GLESSNER ET AL.: 'A genome-wide study
reveals copy number variants exclusive to
childhood obesity cases.' AM J HUM GENET vol.
87, no. 5, 14 October 2010, pages 661 - 666,
XP008157934**

EP 2 539 463 B1

**(Cont. next page)**

- FRAYLING ET AL.: 'A common variant in the FTO gene is associated with body mass index and predisposes to childhood and adult obesity.' SCIENCE vol. 316, no. 5826, 11 May 2007, pages 889 - 894, XP002465136
- AHITUV ET AL.: 'Medical Sequencing at the Extremes of Human Body Mass.' AM J HUM GENET. vol. 80, no. 4, April 2007, pages 779 - 791, XP008157931
- RAJPUT-WILLIAMS ET AL.: 'Variation of apolipoprotein-B gene is associated with obesity, high blood cholesterol levels, and increased risk of coronary heart disease.' LANCET. vol. 332, no. 8626-7, 24 December 1988 - 31 December 1988, pages 1442 - 1446, XP008157629

**Description**

**FIELD OF THE INVENTION**

The present invention relates to the field of obesity. Specifically, the instant invention provides markers for obesity, particularly childhood obesity, and methods of use thereof.

**BACKGROUND OF THE INVENTION**

Several publications and patent documents are cited throughout the specification in order to describe the state of the art to which this invention pertains.

Obesity is a major health problem in modern societies, with increasing prevalence in Western societies, particularly in children (Troiano et al. (1998) Pediatrics, 101:497-504). Obesity and its associated phenotype, insulin resistance (Reaven, G.M. (1988) Diabetes, 37:1595-607; DeFronzo et al. (1991) Diabetes Care, 14:173-94), is also considered a contributor to the major causes of death in the United States and is an important risk factor for type 2 diabetes (T2D), cardiovascular disease (CVD), hypertension and other chronic diseases (National Institutes of Health Consensus Development Conference Statement (1985) Ann. Intern. Med., 103:147-51). Approximately 70% of obese adolescents grow up to become obese adults (Nicklas et al. (2001) J. Am. Coll. Nutr., 20:599-608; Whitaker et al. (1997) N. Engl. J. Med., 337:869-73; Parsons et al. (1999) Int. J. Obes. Relat. Metab. Disord., 23(Suppl 8):S1-107). Indeed, obesity during adolescence has been shown to be associated with increased overall mortality in adults (Must, A. (2003) Nutr. Rev., 61:139-42).

Despite environmental changes over the last 30 years, in particular the unlimited supply of convenient, highly calorific foods together with a sedentary lifestyle, there is also strong evidence for a genetic component to the risk of obesity (Friedman, J.M. (2004) Nat. Med., 10:563-9; Lyon et al. (2005) Am. J. Clin. Nutr., 82:215S-217S). This is reflected in prevalence differences between racial groups (Knowler et al. (1990) Diabetes Metab. Rev., 6:1-27; Zimmet et al. (1990) Diabetes Metab. Rev., 6:91-124). In addition, the familial occurrences of obesity have been long noted with the concordance for fat mass among monozygotic (MZ) twins reported to be 70-90%, higher than the 35-45% concordance in dizygotic (DZ) twins (Stunkard et al. (1986) JAMA 256:51-4; Borjeson et al. (1976) Acta Paediatr. Scand., 65:279-87). Accordingly, the estimated heritability of BMI ranges from 30 to 70% (Hebebrand et al. (2003) Obes. Rev., 4:139-46; Farooqi et al. (2005) Int. J. Obes. (Lond), 29:1149-52; Bell et al. (2005) Nat. Rev. Genet., 6:221-34; Schousboe et al. (2003) Twin Res., 6:409-21).

In the past three years, thirteen genetic loci have been implicated for BMI from the outcomes of genome wide association studies (GWA) studies primarily in adults. Insulin-induced gene 2 (*INSIG2*) was the first locus to be reported by this method to have a role in obesity (Herbert et al. (2006) Science 312:279-83), but replication attempts have yielded inconsistent outcomes (Loos et al. (2007) Science 315:187; Dina et al. (2007) Science 315:187; Rosskopf et al. (2007) Science 315:187; Lyon et al. (2007) PLoS Genet., 3:e61; Hotta et al. (2008) J. Hum. Genet., 53:857-62). The second reported locus, the fat mass- and obesity-associated gene (FTO; Frayling et al. (2007) Science 316:889-94) has been more robustly observed by others (Grant et al. (2008) PLoS ONE 3:e1746; Hinney et al. (2007) PLoS ONE 2:e1361; Dina et al. (2007) Nat. Genet., 39:724-6; Scuteri et al. (2007) PLoS Genet., 3:e115). Subsequent larger studies have uncovered eleven additional loci (Loos et al. (2008) Nat. Genet., 40:768-75; Thorleifsson et al. (2009) Nat. Genet., 41:18-24; Willer et al. (2009) Nat. Genet., 41:25-34). In addition, a copy number variation study of extreme syndromic obesity in children with developmental delay reported a handful of rare variants contributing to the trait (Bochukova et al. (2010) Nature 463:666-70).

**SUMMARY OF THE INVENTION**

In accordance with the present invention, methods of diagnosing an increased risk for obesity in a patient are provided. In a particular embodiment, the methods comprise assessing the presence of at least one copy number variation in a biological sample from the patient. In one embodiment, the copy number variation is in a gene selected from the group consisting of EDIL3, S1PR5, FOXP2, KIF2B, ARL15, and DNAJC15. In one embodiment, the copy number variation is in a gene selected from the group consisting of EDIL3, S1PR5, FOXP2, TBCA, ABCB5, ZPLD1, KIF2B, ARL15, and EPHA6. The copy number variation may be a deletion or duplication.

**BRIEF DESCRIPTIONS OF THE DRAWING**

Figures 1A-1D provide graphs depicting the distributions of the number of copy number variations (CNVs) called per individual of each cohort.

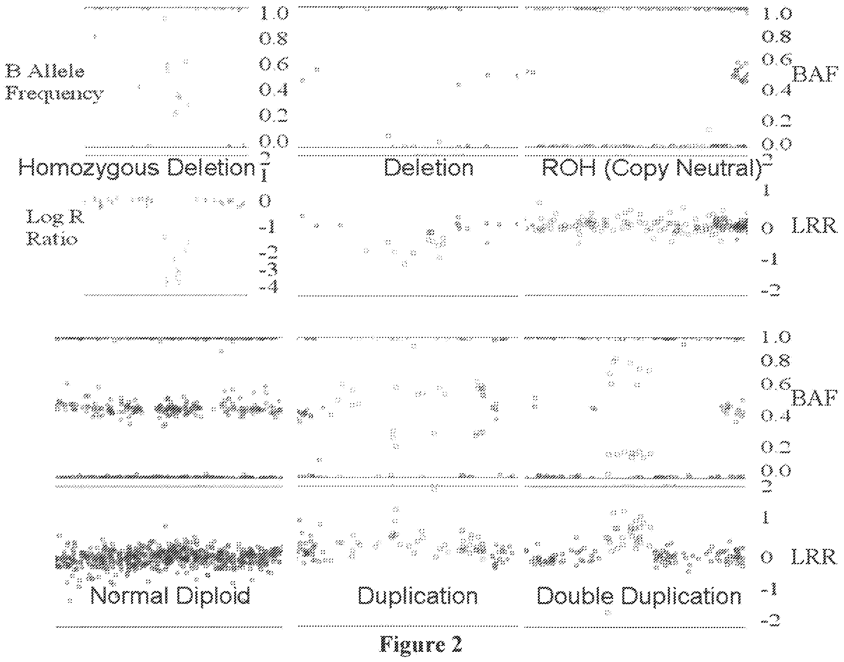

Figure 2 provides examples of CNV observance based on B-allele frequency (BAF) and Log R Ratio (LRR). Distributions of the number of CNVs called per individual of each cohort. LRR is the normalized intensity and BAF is the normalized genotype data. BAF and LRR values are based on the deviation of each sample on each SNP from the cluster center. LRR tends around 0 meaning expected diploid intensity signal. If LRR is elevated, more DNA is being bound indicating there is a duplication. Conversely, LRR decreased signals indicate less DNA is bound, consistent with deletion. In tandem, BAF has three normal diploid modes 0=AA, 0.5=AB, and 1=BB. If 0.5 values are not observed in a contiguous region of SNPs, deletion is probable. If 0.5 values are not observed but 0.33 and 0.66 values are observed, duplication is probable attesting to AAB and ABB genotypes. ROH: a copy number neutral run of homozygosity. The homozygosity is evident in BAF but the LRR does not deviate substantially from zero resulting in a ROH call rather than deletion call.

Figures 3A-3F show loci being directly impacted by deletions or duplications using the UCSC Genome Browser with Build 36 (March 2006) of the human genome. Figure 3A-3C show deletions directly impacting *EDIL3, S1PR5,* and *FOXP2,* respectively. Figures 3D-3F show duplications impacting *KIF2B, DNAJC15,* and *ARL15* respectively.

Figure 4 provides graphs of the quantitative PCR validation of CNVs at the indicated loci. Error bars denote the standard deviation of quadruple runs.

Figure 5 is an analysis flow diagram for statistical informatics of CNV calls from PennCNV (532,898 SNPs utilized on the Illumina® array).

## DETAILED DESCRIPTION OF THE INVENTION

The prevalence of obesity in children and adults in the United States has increased dramatically over the past decade with 30% of the U.S. adult population having BMI above 30 and 5% above 40. A number of genetic determinants of adult obesity have already been established through genome wide association studies and studies of syndromic obesity in childhood. In an attempt to comprehensively identify CNVs conferring susceptibility to regular childhood obesity ($\geq$95th percentile of BMI), a whole-genome CNV study was performed on a cohort of 1,080 childhood obesity cases and 2,500 lean controls (<50th percentile of BMI) of European ancestry who were genotyped with 550,000 SNP markers. Positive findings were evaluated in an independent cohort of 1,479 childhood obesity cases and 1,575 lean controls of African ancestry. 35 CNV loci were identified that were unique in the European American cases, 15 (42.9%) of which also replicated exclusively in African American cases (7 duplications and 8 deletions). 17 CNV loci were identified that were unique to at least three EA cases that both were not previously reported in the public domain and were validated using quantitative PCR. Eight of these loci (47.1%) also replicated exclusively in AA cases (6 deletions and 2 duplications). Replicated deleted loci included *EDIL3* (EGF-like repeats- and discoidin I-like domains-containing protein 3), *S1PR5* (endothelial differentiation, sphingolipid), *FOXP2* (forkhead box P2), TBCA (tubulin folding cofactor A), *ABCB5* (ATP-binding cassette, sub-family B (MDR/TAP), member 5), and *ZPLD1* (zona pellucida-like domain containing 1) while replicated duplications at loci included *KIF2B* (kinesin family member 2B), *AGR3* (breast cancer membrane protein 11 precursor), ARL15 (ADP-ribosylation factor-like 15), and *DNAJC15* (DnaJ homolog, subfamily C, member 15), particularly KIF2B and ARL15. Evidence for a deletion at the EPHA6-UNQ6114 locus was also observed when the AA cohort was investigated as a discovery set. All variants were experimentally validated using quantitative PCR. These variants target genes involved in neurological function, which is an important mediator in the pathogenesis of obesity. These results indicate that CNVs contribute to the genetic susceptibility of obesity in multiple ethnicities.

Definitions

The following definitions are provided to facilitate an understanding of the present invention:

As used herein, the term "obesity" generally refers to a condition in which there is an excess of body fat in a subject. "Obesity" may refer to a condition whereby a subject has a Body Mass Index (BMI; body weight per height in meters squared ($kg/m^2$)) greater than or equal to 30.0 $kg/m^2$. An "obese subject" is a subject with a Body Mass Index (BMI) greater than or equal to 30.0 $kg/m^2$. An "overweight subject" is a subject with a BMI of 25.0 up to 30.0 $kg/m^2$.

The term "copy number", as used herein, refers to the number of copies of a particular region in the genomic DNA of a sample. If a genomic region has a "copy number variation", the genomic region has a copy number that is different than that of the average copy number of the remainder of the genome (e.g., 2 copies in humans).

As used herein, a "biological sample" refers to a sample of biological material obtained from a subject, preferably a human subject, including a tissue, a tissue sample, a cell sample, a tumor sample, and a biological fluid (e.g., blood, urine, or amniotic fluid). In a particular embodiment, the biological sample is blood.

As used herein, "diagnose" refers to detecting and identifying a disease or disorder in a subject. The term may also encompass assessing or evaluating the disease or disorder status (progression, regression, stabilization, response to treatment, etc.) in a patient known to have the disease or disorder.

As used herein, the term "prognosis" refers to providing information regarding the impact of the presence of a disease or disorder (e.g., as determined by the diagnostic methods of the present invention) on a subject's future health (e.g., expected morbidity or mortality, the likelihood of getting diabetes, and the risk of cardiovascular disease). In other words, the term "prognosis" refers to providing a prediction of the probable course and outcome of a disease/disorder or the likelihood of recovery from the disease/disorder.

The term "treat" as used herein refers to any type of treatment that imparts a benefit to a patient afflicted with a disease or disorder, including improvement in the condition of the patient (e.g., in one or more symptoms), delay in the progression of the condition, etc.

The term "probe" as used herein refers to an oligonucleotide, polynucleotide or nucleic acid, either RNA or DNA, whether occurring naturally as in a purified restriction enzyme digest or produced synthetically, which is capable of annealing with or specifically hybridizing to a nucleic acid with sequences complementary to the probe. A probe may be either single-stranded or double-stranded. The exact length of the probe will depend upon many factors, including temperature, source of probe and use of the method. For example, for diagnostic applications, depending on the complexity of the target sequence, the oligonucleotide probe typically contains about 10-100, about 10-50, about 15-30, about 15-25, about 20-50, or more nucleotides, although it may contain fewer nucleotides. The probes herein may be selected to be complementary to different strands of a particular target nucleic acid sequence. This means that the probes must be sufficiently complementary so as to be able to "specifically hybridize" or anneal with their respective target strands under a set of pre-determined conditions. Therefore, the probe sequence need not reflect the exact complementary sequence of the target, although they may. For example, a non-complementary nucleotide fragment may be attached to the 5' or 3' end of the probe, with the remainder of the probe sequence being complementary to the target strand. Alternatively, non-complementary bases or longer sequences can be interspersed into the probe, provided that the probe sequence has sufficient complementarity with the sequence of the target nucleic acid to anneal therewith specifically.

The term "primer" as used herein refers to an oligonucleotide, either RNA or DNA, either single-stranded or double-stranded, either derived from a biological system, generated by restriction enzyme digestion, or produced synthetically which, when placed in the proper environment, is able to functionally act as an initiator of template-dependent nucleic acid synthesis. When presented with an appropriate nucleic acid template, suitable nucleoside triphosphate precursors of nucleic acids, a polymerase enzyme, suitable cofactors and conditions such as appropriate temperature and pH, the primer may be extended at its 3' terminus by the addition of nucleotides by the action of a polymerase or similar activity to yield a primer extension product. The primer may vary in length depending on the particular conditions and requirement of the application. For example, in diagnostic applications, the oligonucleotide primer is typically about 10-25 or more nucleotides in length. The primer must be of sufficient complementarity to the desired template to prime the synthesis of the desired extension product, that is, to be able to anneal with the desired template strand in a manner sufficient to provide the 3' hydroxyl moiety of the primer in appropriate juxtaposition for use in the initiation of synthesis by a polymerase or similar enzyme. It is not required that the primer sequence represent an exact complement of the desired template. For example, a non-complementary nucleotide sequence may be attached to the 5' end of an otherwise complementary primer. Alternatively, non-complementary bases may be interspersed within the oligonucleotide primer sequence, provided that the primer sequence has sufficient complementarity with the sequence of the desired template strand to functionally provide a template-primer complex for the synthesis of the extension product.

Polymerase chain reaction (PCR) has been described in U.S. Patents 4,683,195, 4,800,195, and 4,965,188.

With respect to single stranded nucleic acids, particularly oligonucleotides, the term "specifically hybridizing" refers to the association between two single-stranded nucleotide molecules of sufficiently complementary sequence to permit such hybridization under pre-determined conditions generally used in the art (sometimes termed "substantially complementary"). In particular, the term refers to hybridization of an oligonucleotide with a substantially complementary sequence contained within a single-stranded DNA molecule of the invention, to the substantial exclusion of hybridization of the oligonucleotide with single-stranded nucleic acids of non-complementary sequence. Appropriate conditions enabling specific hybridization of single stranded nucleic acid molecules of varying complementarity are well known in the art.

For instance, one common formula for calculating the stringency conditions required to achieve hybridization between nucleic acid molecules of a specified sequence homology is set forth below (Sambrook et al., 1989, Molecular Cloning: A Laboratory Manual, Cold Spring Harbor Laboratory Press):

$$Tm = 81.5°C + 16.6Log\ [Na+] + 0.41(\%\ G+C) - 0.63\ (\%\ formamide) - 600/\#bp\ in\ duplex$$

As an illustration of the above formula, using [Na+] = [0.368] and 50% formamide, with GC content of 42% and an average probe size of 200 bases, the Tm is 57°C. The Tm of a DNA duplex decreases by 1 - 1.5°C with every 1% decrease in homology. Thus, targets with greater than about 75% sequence identity would be observed using a hybridization temperature of 42°C.

The stringency of the hybridization and wash depend primarily on the salt concentration and temperature of the solutions. In general, to maximize the rate of annealing of the probe with its target, the hybridization is usually carried out at salt and temperature conditions that are 20-25°C below the calculated Tm of the hybrid. Wash conditions should be as stringent as possible for the degree of identity of the probe for the target. In general, wash conditions are selected to be approximately 12 20°C below the Tm of the hybrid. In regards to the nucleic acids of the current invention, a moderate stringency hybridization is defined as hybridization in 6X SSC, 5X Denhardt's solution, 0.5% SDS and 100 μg/ml denatured salmon sperm DNA at 42°C, and washed in 2X SSC and 0.5% SDS at 55°C for 15 minutes. A high stringency hybridization is defined as hybridization in 6X SSC, 5X Denhardt's solution, 0.5% SDS and 100 μg/ml denatured salmon sperm DNA at 42°C, and washed in 1X SSC and 0.5% SDS at 65°C for 15 minutes. A very high stringency hybridization is defined as hybridization in 6X SSC, 5X Denhardt's solution, 0.5% SDS and 100 μg/ml denatured salmon sperm DNA at 42°C, and washed in 0.1X SSC and 0.5% SDS at 65°C for 15 minutes.

The term "isolated" may refer to a compound or complex that has been sufficiently separated from other compounds with which it would naturally be associated. "Isolated" is not meant to exclude artificial or synthetic mixtures with other compounds or materials, or the presence of impurities that do not interfere with fundamental activity or ensuing assays, and that may be present, for example, due to incomplete purification, or the addition of stabilizers.

As used herein, an "instructional material" includes a publication, a recording, a diagram, or any other medium of expression which can be used to communicate the usefulness of the composition of the invention for performing a method of the invention.

The phrase "solid support" refers to any solid surface including, without limitation, any chip (for example, silica-based, glass, or gold chip), glass slide, membrane, plate, bead, solid particle (for example, agarose, sepharose, polystyrene or magnetic bead), column (or column material), test tube, or microtiter dish.

As used herein, the term "array" refers to an ordered arrangement of hybridizable array elements (e.g., proteins, nucleic acids, antibodies, etc.). The array elements are arranged so that there are at least one or more different array elements on a solid support. In a particular embodiment, the array elements comprise oligonucleotide probes.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, excipient, auxilliary agent or vehicle with which an active agent of the present invention is administered. Pharmaceutically acceptable carriers can be sterile liquids, such as water and oils, including those of petroleum, animal, vegetable or synthetic origin, such as peanut oil, soybean oil, mineral oil, sesame oil and the like. Water or aqueous saline solutions and aqueous dextrose and glycerol solutions are preferably employed as carriers, particularly for injectable solutions. Suitable pharmaceutical carriers are described in "Remington's Pharmaceutical Sciences" by E.W. Martin (e.g., Remington's Pharmaceutical Sciences, 18th Ed. (1990, Mack Publishing Co., Easton, PA 18042)) and "Remington: The Science and Practice of Pharmacy" (Ed. Troy; Lippincott Williams & Wilkins, Baltimore, MD).

### Diagnostic Methods, Kits, and Screening Methods

The instant invention provides methods of diagnosing and/or determining the susceptibility to/risk for obesity in a subject (e.g., mammal, human). The methods may further comprise providing a prognosis for related health problems. If a subject is diagnosed as being at risk for obesity, the subject's diet may be altered to avoid/lessen/treat obesity; the subject may be administered therapeutic agents to prevent/treat obesity; and/or the subject may be provided an exercise regimen.

In one embodiment, the method comprises determining the genomic copy number of at least one loci provided in Table 3, 4, or 7 in a biological sample obtained from a patient. In another embodiment, the method comprises determining the copy number of at least one gene selected from the group consisting of EPHA6, EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, and RPAP3 in a biological sample obtained from a patient.

In another embodiment, the gene is selected from the group consisting of S1PR5, FOXP2, TBCA, ABCB5, PNLIPRP1, BC015432, CDS2, CACNA2D4, LRTM2, CENTD1, BC073935, CR611653, DNAJC15, AGR3, DFNB31, COL27A1, AKNA, ATP6V1G1, ORM1, ORM2, C9orf91, ARL15, PKD1L2, BCMO1, MICB, COMT, NXPH1, GPR98, BC048997, and AK091626.

In yet another embodiment, the gene is selected from the group consisting of EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91, and ARL15.

In still another embodiment, the gene is selected from the group consisting of EDIL3, S1PR5, FOXP2, KIF2B, ARL15, and DNAJC15. In still another embodiment, the gene is selected from the group consisting of EDIL3, S1PR5, FOXP2, TBCA, ABCB5, ZPLD1, KIF2B, and ARL15.

In a particular embodiment, when the gene is selected from the group consisting of EPHA6, EDIL3, S1PR5, FOXP2, TBCA, ABCB5, ZPLD1, DERA, PNLIPRP1, PRR20A, BC015432, CDS2, CACNA2D4, LRTM2, and CENTD1, the copy number variation indicative of obesity is a deletion.

In another embodiment, when the gene is selected from the group consisting of KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, and RPAP3, the copy number variation indicative of obesity is a duplication.

The NCBI GeneID Nos. and GenBank Accession Nos. (which provide nucleotide and amino acid sequences) of the above genes are:

> EDIL3 (EGF-like repeats and discoidin I-like domains 3): GeneID: 10085
> S1PR5 (sphingosine-1-phosphate receptor 5; also known as EDG8): GeneID: 53637
> FOXP2 (forkhead box P2): GeneID: 93986
> TBCA (tubulin folding cofactor A): GeneID: 6902
> ABCB5 (ATP-binding cassette, sub-family B (MDR/TAP), member 5): GeneID: 340273
> ZPLD1 (zona pellucida-like domain containing 1): GeneID: 131368
> DERA (2-deoxyribose-5-phosphate aldolase homolog): GeneID: 51071
> PNLIPRP1 (pancreatic lipase-related protein 1): GeneID: 5407
> PRR20A (proline rich 20A; also known as FLJ40296): GeneID: 122183
> BC015432: GenBank Accession No. BC015432.1
> CDS2 (CDP-diacylglycerol synthase (phosphatidate cytidylyltransferase) 2): GeneID: 8760
> CACNA2D4 (calcium channel, voltage-dependent, alpha 2/delta subunit 4): GeneID: 93589
> LRTM2 (leucine-rich repeats and transmembrane domains 2): GeneID: 654429
> CENTD1 (centaurin, delta 1; also known as ARAP2): GeneID: 116984
> KIF2B (kinesin family member 2B): GeneID: 84643
> BC073935: GenBank Accession No. BC073935.1
> CR611653: GenBank Accession No. CR611653.1
> BRDT (bromodomain, testis-specific): GeneID: 676
> DNAJC15 (DnaJ (Hsp40) homolog, subfamily C, member 15): GeneID: 29103
> AGR3 (anterior gradient homolog 3): GeneID: 155465
> DFNB31 (deafness, autosomal recessive 31): GeneID: 25861
> COL27A1 (collagen, type XXVII, alpha 1): GeneID: 85301
> AKNA (AT-hook transcription factor): GeneID: 80709
> ATP6V1G1 (ATPase, H+ transporting, lysosomal 13kDa, V1 subunit G1) : GeneID: 9550
> ORM1 (orosomucoid 1): GeneID: 5004
> ORM2 (orosomucoid 2): GeneID: 5005
> C9orf91 (chromosome 9 open reading frame 91): GeneID: 203197
> ARL15 (ADP-ribosylation factor-like 15): GeneID: 54622
> BCMO1 (beta-carotene 15,15'-monooxygenase 1; also known as BCDO): GeneID: 53630
> PKD1L2 (polycystic kidney disease 1-like 2): GeneID: 114780
> MICB (MHC class I polypeptide-related sequence B): GeneID: 4277
> COMT (catechol-O-methyltransferase): GeneID: 1312
> SLCO1A2 (solute carrier organic anion transporter family, member 1A2) : GeneID: 6579
> NXPH1 (neurexophilin 1): GeneID: 30010
> EGFL11 (EGF-like-domain, multiple 11; also known as EYS): GeneID: 346007
> NAALADL2 (N-acetylated alpha-linked acidic dipeptidase-like 2): GeneID: 254827
> GPR98 (G protein-coupled receptor 98; also known as KIAA0686): GeneID: 84059
> PCDH20 (protocadherin 20): GeneID: 64881
> BC048997: GenBank Accession No. BC048997.1
> AK091626: GenBank Accession No. AK091626.1
> APOB (apolipoprotein B): GeneID: 338
> RPAP3 (RNA polymerase II associated protein 3): GeneID: 79657

EPHA6 (ephrin receptor A6; UNQ6114; MIM 600066): Gene ID: 285220

Copy number variation can be detected by any method known in the art. For example, copy number variation may be detected by PCR (e.g., qPCR), fluorescent in situ hybridization (FISH), comparative genomic hybridization, array comparative genomic hybridization, and virtual karyotyping.

Probes and primers which specifically hybridize at least one of the genes of the instant invention are also encompassed by the instant invention. The probes and primers may be used to determine the copy number of the genes. The probes may be immobilized on a solid support. Indeed, arrays comprising at least one probe for at least one, at least two, at least six, or all of the genes of the instant invention are also encompassed by the instant invention. In a particular embodiment, the array comprises at least one probe for each gene in the above-identified groups.

The probes, primers, and/or arrays of the instant invention may be incorporated into a kit. The kit may further comprise instruction material, buffers, and/or containers.

In addition to the above, the instant invention encompasses methods for screening (including high throughput screening) for therapeutic agents for treating, inhibiting, and/or preventing obesity. Since the CNVs identified herein have been associated with the etiology of obesity, methods for identifying agents that modulate the activity of the genes and their encoded products (e.g., restore to normal levels) will result in the generation of efficacious therapeutic agents (e.g., nucleic acids (e.g., DNA, cDNA, RNA, siRNA, antisense, etc.), proteins, polypeptides, ligands, antibodies, small molecules, etc.) for the treatment of this condition. In a particular embodiment, the agents to be tested were developed by rational drug design. As an example, for CNVs wherein there is a deletion of a gene and concomitant loss in activity in the gene and/or gene product (e.g., those provided in the above GeneID references), therapeutic agents which increase activity levels (e.g., to wild-type levels) would be desirable. Additionally, for CNVs wherein a gene is duplicated and there is a concomitant increase in activity in the gene and/or gene product, therapeutic agents which decrease activity levels (e.g., to wild-type levels) would be desirable.

In a particular embodiment, the screening methods comprise contacting cells comprising at least one CNV of the instant invention with a compound and assessing whether an activity of the gene and/or gene product affected by the CNV is modulated (e.g., closer to wild-type), thereby identifying a therapeutic agent. In a particular embodiment, the cells are obtained from a subject. In yet another embodiment, the CNVs are generated in a cell in vitro. Methods for modulating the CN of a gene or locus in a cell are well known in the art. In still another embodiment, the screening methods may be performed on an animal (e.g., a transgenic animal (e.g., mouse)) comprising at least one CNV of the instant invention.

The elucidation of the role played by the CNVs described herein in cellular metabolism facilitates the development of pharmaceutical compositions useful for treatment and diagnosis of obesity. These compositions may comprise, in addition to one of the above substances, a pharmaceutically acceptable carrier (e.g., excipient, carrier, buffer, stabilizer or other materials well known to those skilled in the art). Such materials should be non-toxic and should not interfere with the efficacy of the active ingredient. Whether it is a polypeptide, antibody, peptide, nucleic acid molecule, small molecule or other pharmaceutically useful compound according to the present invention that is to be given to an individual, administration is preferably in a "prophylactically effective amount" or a "therapeutically effective amount" (as the case may be, although prophylaxis may be considered therapy), this being sufficient to show benefit to the individual.

In yet another embodiment, the instant invention encompasses pharmacogenomics. More specifically, the instant invention encompasses methods of screening a subject to predict the efficacy of a therapeutic agent or to select the most effective therapeutic agent for treatment. In a particular embodiment, the method comprises obtaining a biological sample from a patient and determining the presence or absence of at least one CNV of the instant invention. The subject is then administered the therapeutic agent if it is demonstrated that the administration of the therapeutic agent to a subject with the CNV is efficacious and without unacceptable side effects.

The following example provides illustrative methods of practicing the instant invention, and is not intended to limit the scope of the invention in any way.

## EXAMPLE

## METHODS

*Study Subjects*

All subjects were consecutively recruited from the Greater Philadelphia area from 2006 to 2009 at the Children's Hospital of Philadelphia. The study consisted of 1,080 Caucasian obese children (BMI ≥ 95th percentile), 2,500 Caucasian lean controls (BMI < 50th percentile), 1,479 AA obese children and 1,575 AA lean controls that met strictly established data quality thresholds for CNVs. All of these participants had their blood drawn in to a 6 ml EDTA blood collection tube and were subsequently DNA extracted for genotyping. BMI ≥ 95th percentile was defined using the Center for Disease control (CDC) z-score = 1.645 (www.cdc.gov/nchs/about/major/nhanes/growthcharts/datafi les.htm). All subjects were biologically unrelated (based on available SNP data (pairwise IDB was used to filter out individuals showing relatedness above 0.25) and were aged between 2 and 18 years old (Table 1). All subjects were between -3 and +3 standard deviations of CDC corrected BMI (i.e., outliers were excluded to avoid the consequences of potential measurement error or Mendelian causes of extreme obesity; the latter category therefore specifically excluded individuals that fall in to the category previously reported for CNV discovery (Bochukova et al. (2010) Nature 463:666-70; Walters et al. (2010) Nature 463:671-5)). Self-reported ethnicity was confirmed by multidimensional scaling methodologies. Global ancestry was determined using Eigenstrat to ensure a singular cluster of ethnic ancestry in the European and African populations. This study was approved by the Institutional Review Board of the Children's Hospital of Philadelphia. Parental informed consent was given for each study participant for both the blood collection and subsequent genotyping.

**Table 1:** Gender and age distribution in the cohorts studied.

| Cohort | Age mean (SD) | Gender (Male Frequency) |
| --- | --- | --- |
| Caucasian Obese | 10.3 (4.47) | 0.57 |
| Caucasian Lean | 9.10 (5.21) | 0.54 |
| African Obese | 10.4 (4.72) | 0.48 |
| African Lean | 7.11 (5.48) | 0.52 |

*Illumina® Infinium assay for CNV Discovery*

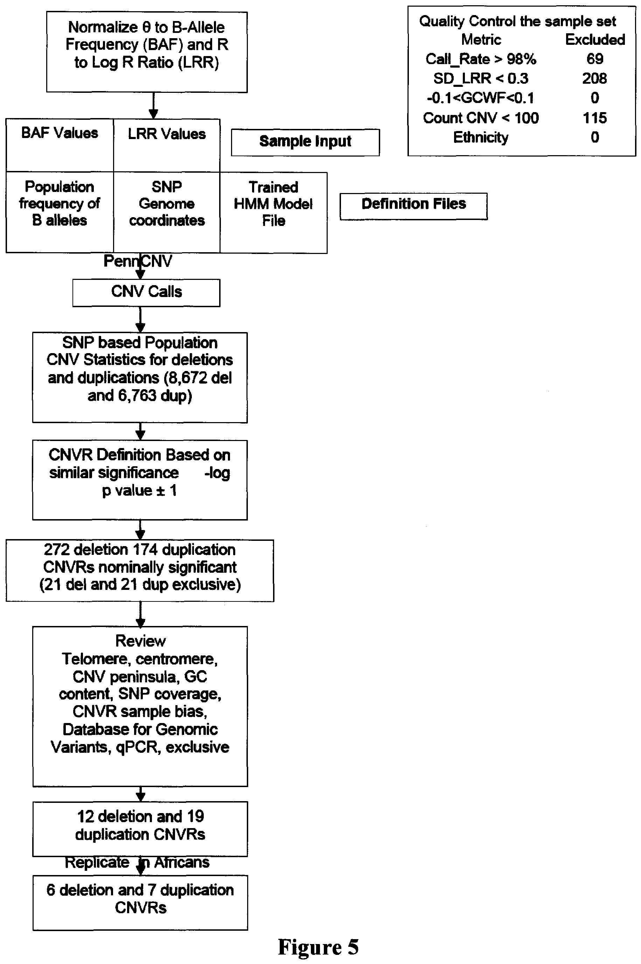

High-throughput, genome-wide SNP genotyping was performed using the Infinium™ II HumanHap550 Bead-Chip technology (Illumina®; San Diego, CA), at the Center for Applied Genomics at the Children's Hospital of Philadelphia. The genotype data content together with the intensity data provided by the genotyping array provides high confidence for CNV calls. Importantly, the simultaneous analysis of intensity data and genotype data in the same experimental setting establishes a highly accurate definition for normal diploid states and any deviation thereof. To call CNVs, the PennCNV algorithm (www.openbioinformatics.org/penncnv/) was used, which combines multiple sources of information, including Log R Ratio (LRR) and B Allele Frequency (BAF) at each SNP marker, along with SNP spacing, a trained hidden Markov model, and population frequency of the B allele to generate CNV calls. Each sample had CNVs called blinded to case status. Positive findings were evaluated in an independent replication cohort of similar size of African ancestry. A flow diagram of the CNV filtering and testing steps is provided in Figure 5.

*CNV quality control*

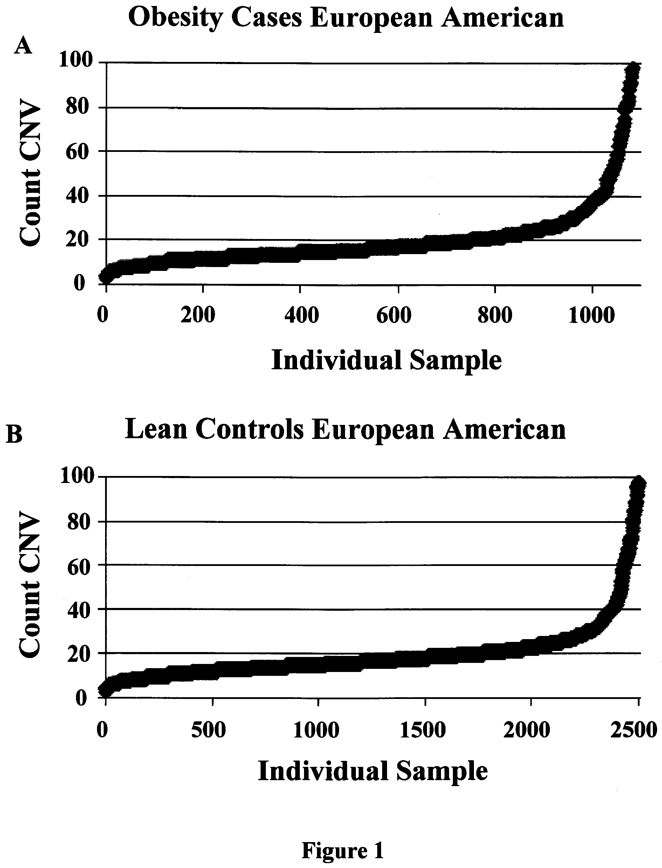

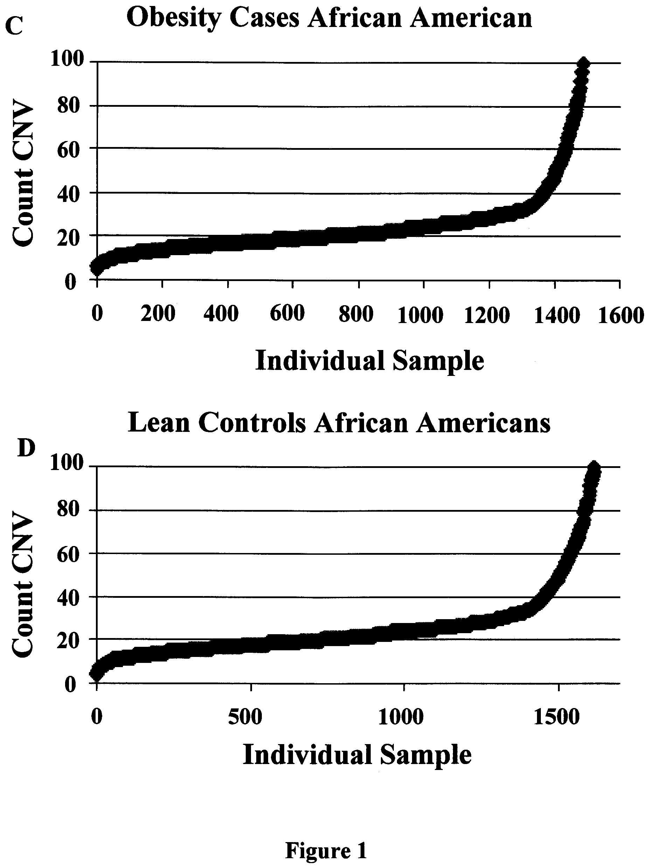

Quality Control (QC) measures was calculated on the HumanHap550 GWAS data based on statistical distributions to exclude poor quality DNA samples and false positive CNVs. The first threshold is the percentage of attempted SNPs which were successfully genotyped. Only samples with call rate > 98% were included. The genome wide intensity signal must have as little noise as possible. Only samples with the standard deviation (SD) of normalized intensity (LRR) < 0.30 were included. All samples must have clear Caucasian or African ethnicity based on Multiple Dimension Scaling (MDS) scoring and all other samples were excluded. Wave artifacts roughly correlating with GC content resulting from hybridization bias of low full length DNA quantity are known to interfere with accurate inference of copy number variations (Diskin et al. (2008) Nucleic Acids Res., 36:e126). Only samples where the GC wave factor of LRR to wave model ranged between $-0.1<X<0.1$ were accepted. If the count of CNV calls made by PennCNV exceeds 100 (Figure 1), the DNA quality is usually poor. Thus, only samples with CNV call count < 100 were included. Any duplicate samples (such as monozygotic twins) had one sample excluded.

*Statistical analysis of CNVs*

CNV frequency between cases and controls was evaluated at each SNP using Fisher's exact test. Only loci that were significant between cases and controls (p<0.05) where cases in the European American discovery cohort had the same variation, replicated in African Americans or were not observed in any of the control subjects, and validated with an independent method were considered. Statistical local minimums are reported to narrow the association in reference to a region of nominal significance including SNPs residing within 1 Mb of each other. Resulting significant CNVRs were excluded if they met any of the following criteria: i) residing on telomere or centromere proximal cytobands; ii) arising in a "peninsula" of common CNV arising from variation in boundary truncation of CNV calling; iii) genomic regions with extremes in GC content which produces hybridization bias; or iv) samples contributing to multiple CNVRs. DAVID (Database for Annotation, Visualization, and Integrated Discovery)(Dennis et al. (2003) Genome Biol., 4:P3;

Huang da et al. (2007) Genome Biol., 8:R183) was used to assess the significance of functional annotation clustering of independently associated CNV results into InterPro categories (the top result is UniProt Category: Vision P=0.0069 (CACNA2D4, GPR98, DFNB31). To adjust for number of tests performed, correction of 16 deletion and 19 duplication CNVRs was made based on significance in the European American cohort.

*CNV Filtering Steps*

Multiple CNV filtering steps have been performed as part of the analysis. Firstly, it is important to note that of the 532,898 SNPs on the Illumina array, 8,672 (1.627%) showed deletion and 6,763 (1.269%) showed duplication in at least three or more unrelated cases in the European American discovery cohort (frequency $\geq$ 0.278%). The threshold of three cases is selected because it is the minimal case frequency to provide reproducibility for the calls in a given region. This upfront exclusion is very similar to the inclusion threshold of 1% Minor Allele Frequency in GWA SNP genotype studies. This drastically cuts down on the number of test preformed to correct for genome wide testing.

Secondly, all CNVs were called simultaneously in both cases and controls and classified into CNVRs as defined in the manuscript. A total of 272 deletion and 174 duplication CNVRs were identified. Thirdly, to search for novel CNVs, all CNVRs observed in the Caucasian control cohort were filtered out to establish exclusivity in cases and carefully reviewed the raw data (BAF and LRR) for accurate CNV calling and statistical significance as described in Methods. This left 12 deletion and 19 duplication CNVRs.

This dataset therefore defines the CNVRs from the discovery cohort that were used to test for novel Obesity CNVs. Replication of these CNVRs was then tested in the independent African American case-control dataset. 6 deletion and 7 duplication CNVRs survived the replication criteria (observed in an African American case and absent in the African American control set) and were subsequently experimentally validated with at least one independent method (QPCR and FISH). These results are shown in Table 3.

It is important to note that CNV calling is not unequivocally attained by any one platform for multiple reasons, including variations in DNA provided, array type, DNA processing, data processing, quality control, CNV calling algorithm, genomic features, genomic coverage and statistical presentation of regions. This can lead to high false positive rate upon initial inspection despite exhaustive efforts to standardize and control each confounding contribution.

*CNV validation by quantitative PCR (QPCR)*

Universal Probe Library (UPL; Roche, Indianapolis, IN) probes were selected using the ProbeFinder v2.41 software (Roche, Indianapolis, IN). Quantitative PCR was performed on an ABI 7500 Real Time PCR Instrument or on an ABI Prism™ 7900HT Sequence Detection System (Applied Biosystems, Foster City, CA). Each sample was analyzed in quadruplicate either in 25 $\mu$l reaction mixture (250 nM probe, 900 nM each primer, Fast Start TaqMan Probe Master from Roche, and 10 ng genomic DNA) or in 10 $\mu$l reaction mixture (100 nM probe, 200 nM each primer, 1x Platinum Quantitative PCR SuperMix-Uracil-DNA-Glycosylase (UDG) with ROX from Invitrogen, and 25 ng genomic DNA). The values were evaluated using Sequence Detection Software v2.2.1 (Applied Biosystems, CA). Data analysis was further performed using the $\Delta\Delta C_T$ method. Reference genes, chosen from *COBL* (MIM 610317), *GUSB* (MIM 611499), and *SNCA* (MIM 163890), were included based on the minimal coefficient of variation and then data was normalized by setting a normal control to a value of 1. None of these rare CNVs could be effectively tagged by a single common SNP present on the array (Table 2).

**Table 2:** Best SNP genotype surrogates for the CNVs and their respective $r^2$ values, reflecting the level of linkage disequilibrium between the markers. Due to the common nature of SNPs >1% and the rare nature of CNVs <1%, it is challenging to identify SNP genotype surrogates for the CNVs. Most $r^2$ values of genotyped SNPs within 1Mb of the CNV were close to zero.

| CNV | SNP | $r^2$ |
| --- | --- | --- |
| chr5:83835179-83874339 | rs13355569 | 0.012672 |
| chr5:83835179-83874339 | rs10041822 | 0.00577 |
| chr19:10489548-10512171 | rs7256672 | 0.000808 |
| chr19:10489548-10512171 | rs281423 | 0.000599 |
| chr7:113843696-113859679 | rs10255943 | 0.001823 |
| chr7:113843696-113859679 | rs7782412 | 0.001009 |
| chr5:77039051-77076628 | rs2359877 | 0.001682 |
| chr5:77039051-77076628 | rs2359875 | 0.001568 |
| chr7:20708193-20711088 | rs1108864 | 0.001978 |

(continued)

| CNV | SNP | r$^2$ |
|---|---|---|
| chr7:20708193-20711088 | rs6954985 | 0.001187 |
| chr3:104059109-104092618 | rs6787298 | 0.001848 |
| chr3:104059109-104092618 | rs938988 | 0.001083 |
| chr12:16141980-16149714 | rs6488792 | 0.003442 |
| chr12:16141980-16149714 | rs4763429 | 0.002848 |
| chr10:118354808-118363319 | rs4751986 | 0.001028 |
| chr10:118354808-118363319 | rs17095060 | 0.000927 |
| chr13:56772821-56786987 | rs9537876 | 0.026849 |
| chr13:56772821-56786987 | rs1475441 | 0.02579 |
| chr20:5111828-5137472 | rs17785402 | 0.003542 |
| chr20:5111828-5137472 | rs6038092 | 0.0034 |
| chr12:1778115-1803814 | rs11608641 | 0.001571 |
| chr12:1778115-1803814 | rs11611260 | 0.001276 |
| chr4:35782722-35828015 | rs6826501 | 0.000984 |
| chr4:35782722-35828015 | rs4833119 | 0.000724 |
| chr17:49444406-49449022 | rs807101 | 0.001083 |
| chr17:49444406-49449022 | rs9303339 | 0.00103 |
| chr12:8322269-8401135 | rs2058456 | 0.001399 |
| chr12:8322269-8401135 | rs10161484 | 0.001034 |
| chr1:92164066-92168702 | rs17131553 | 0.000749 |
| chr1:92164066-92168702 | rs643927 | 0.00062 |
| chr13:42467085-42516530 | rs9590722 | 0.001425 |
| chr13:42467085-42516530 | rs10492434 | 0.001386 |
| chr7:16885697-17020565 | rs7809828 | 0.001501 |
| chr7:16885697-17020565 | rs6949072 | 0.000957 |
| chr9:115996089-116495996 | rs1003859 | 0.004612 |
| chr9:115996089-116495996 | rs11999545 | 0.001678 |
| chr5:53467427-53480255 | rs271246 | 0.002212 |
| chr5:53467427-53480255 | rs12153556 | 0.001691 |
| chr16:79761583-79838201 | rs7199318 | 0.005738 |
| chr16:79761583-79838201 | rs8058311 | 0.005555 |
| chr6:31567721-31580699 | rs2596480 | 0.003791 |
| chr6:31567721-31580699 | rs2267637 | 0.003166 |
| chr22:17405381-19770502 | rs5748302 | 0.010889 |
| chr22:17405381-19770502 | rs16984071 | 0.001003 |
| chr12:21446005-21459060 | rs4762726 | 0.001848 |
| chr12:21446005-21459060 | rs10841939 | 0.00179 |
| chr7:8710455-8984816 | rs6962574 | 0.00223 |
| chr7:8710455-8984816 | rs10239677 | 0.001396 |
| chr6:67391945-67395314 | rs16897804 | 0.002653 |
| chr6:67391945-67395314 | rs7767899 | 0.001389 |
| chr3:177280907-177303744 | rs1461249 | 0.00236 |
| chr3:177280907-177303744 | rs12488992 | 0.002253 |
| chr5:90155080-90157263 | rs2914673 | 0.001697 |
| chr5:90155080-90157263 | rs1809814 | 0.00102 |
| chr13:61409439-61413267 | rs3121782 | 0.002359 |
| chr13:61409439-61413267 | rs9539012 | 0.001593 |
| chr13:22255136-22381716 | rs314862 | 0.001881 |
| chr13:22255136-22381716 | rs17077897 | 0.001741 |

(continued)

| CNV | SNP | $r^2$ |
|---|---|---|
| chr2:21337060-21342588 | rs11096672 | 0.001064 |
| chr2:21337060-21342588 | rs10198972 | 0.000911 |
| chr12:46232064-46330773 | rs2087343 | 0.00186 |
| chr12:46232064-46330773 | rs4760607 | 0.001848 |

**RESULTS**

The instant study consisted of 1,080 Caucasian obese children (BMI $\geq$ 95th percentile), 2,500 Caucasian lean controls (BMI < 50th percentile), 1,479 AA obese children and 1,575 AA lean controls (2-18 years old) that met strictly established data quality thresholds for CNVs. However, all subjects had to be between -3 and +3 standard deviations of CDC corrected BMI in order to exclude outliers that could potentially be a result of measurement error or Mendelian causes of extreme obesity. Indeed, this latter category specifically excludes individuals that fall in to the category previously reported for CNV discovery (Bochukova et al. (2010) Nature 463:666-70; Walters et al. (2010) Nature 463:671-5).

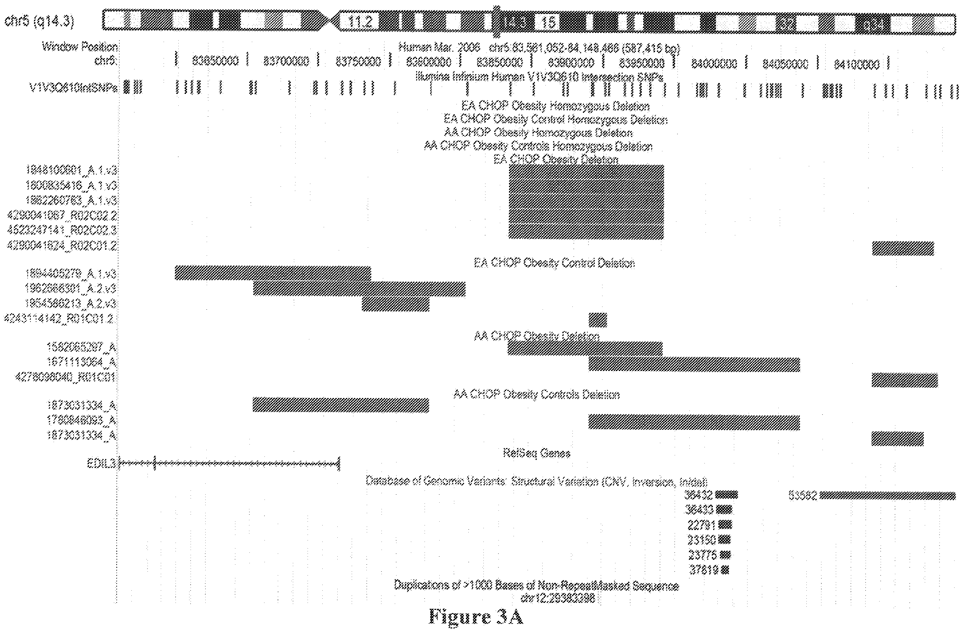

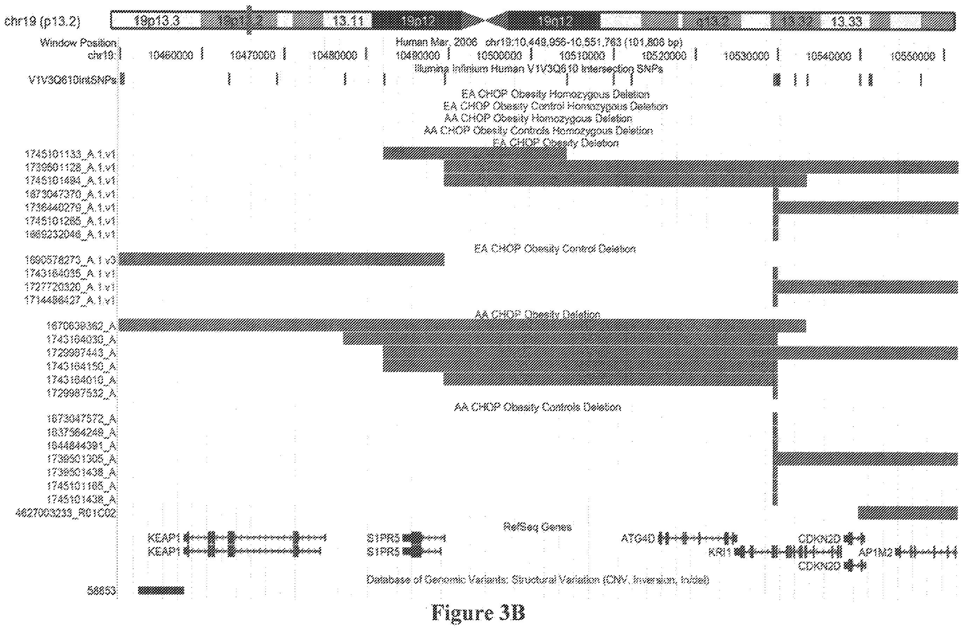

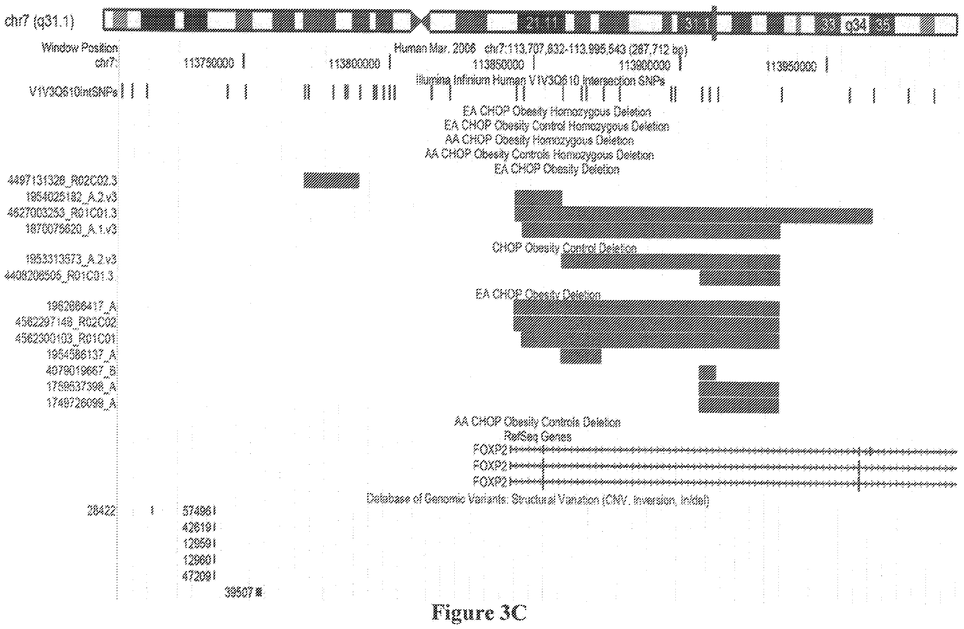

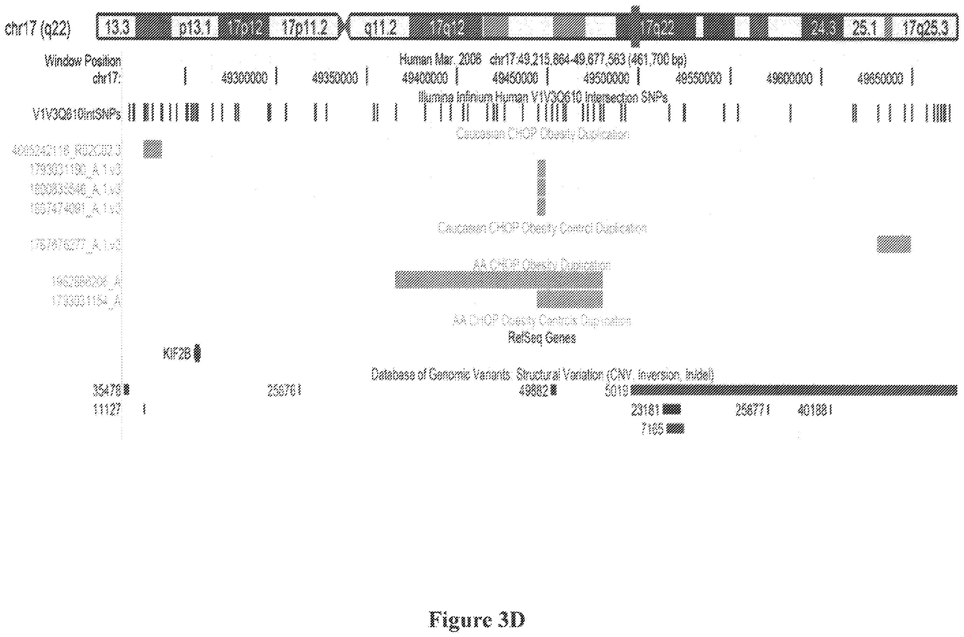

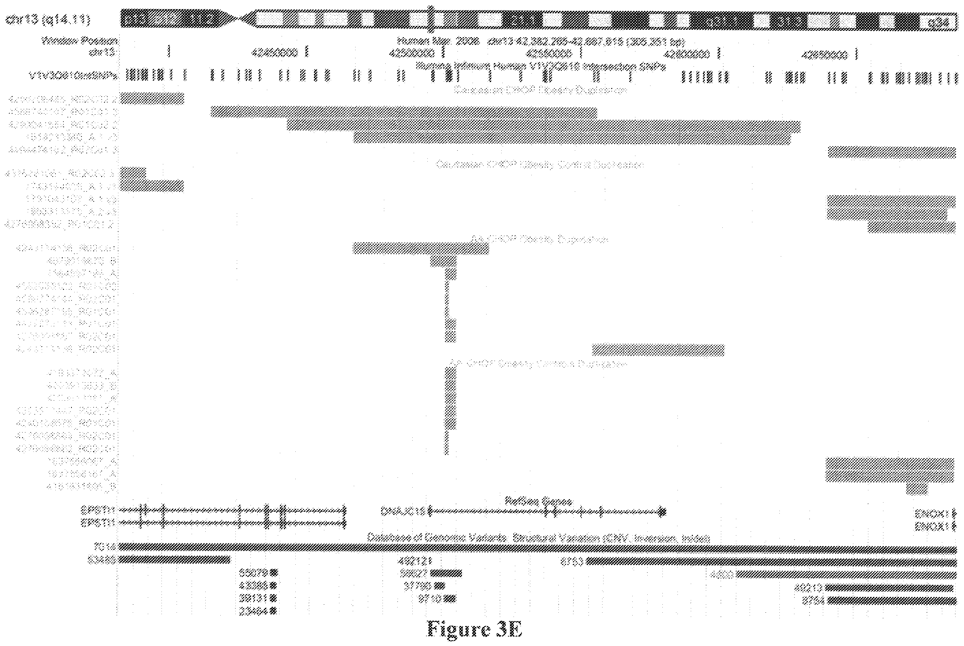

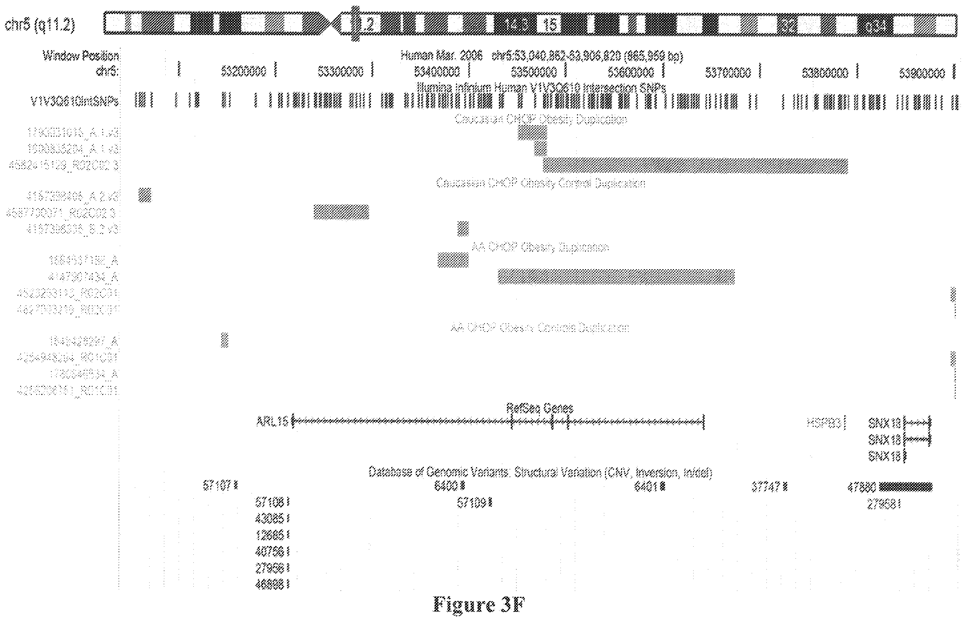

An average of 22.0 and 19.5 CNV calls per individual were made using the PennCNV software (Wang et al. (2007) Genome Res., 17:1665-74) in the European American (EA) cases and controls, respectively, with 93% of subjects having 8-45 CNV calls (Figure 3). Four different copy number states were called for EA case/control cohorts, including 919/2,109 homozygous deletions (copy number, or CN =0), 13,678/28,917 hemizygous deletions (CN =1), 6,767/16,689 one copy duplications (CN =3), and 344/1,139 two copy duplications (CN =4), respectively. Figure 2 shows an example of raw Illumina data as viewed in the BeadStudio software and the resulting CNV call. The CNV calls spanned from 3 to 7903 SNPs, with an average of 19 SNPs per CNV call, and their sizes ranged from 50bp to 33Mb, with an average size of 81kb.

93% of African American (AA) subjects also harbored 8-45 CNV calls (Figure 1). An average of 24.1 and 25.1 CNV calls per individual were made in AA cases and controls, respectively. Among them, 897/1,026 CN =0, 25,650/28,793 CN =1, 8,778/10,251 CN =3 and 418/535 CN =4 were identified. The CNV calls spanned from 3 to 7903 SNPs, with an average of 15 SNPs per CNV call, and their sizes ranged from 50bp to 33Mb, with an average size of 67kb.

To identify novel genomic loci potentially contributing to non-syndromic childhood obesity in the EA subjects, a segment-based scoring approach was applied that scans the genome for consecutive SNPs with more frequent copy number changes in cases compared to controls. The genomic span for these consecutive SNPs delineates common copy number variation regions, or CNVRs.

Local ancestry was assessed using the 1MB region surrounding each CNV locus which included an average of 300 SNP genotypes, resulting in well clustered populations without significantly deviating individuals. Statistical local minimums are reported to narrow the association in reference to a region of nominal significance including SNPs residing within 1 Mb of each other. Resulting significant CNVRs were excluded if they met any of the following criteria: i) residing on telomere or centromere proximal cytobands; ii) arising in a "peninsula" of common CNV arising from variation in boundary truncation of CNV calling; iii) genomic regions with extremes in GC content which produces hybridization bias; or iv) samples contributing to multiple CNVRs.

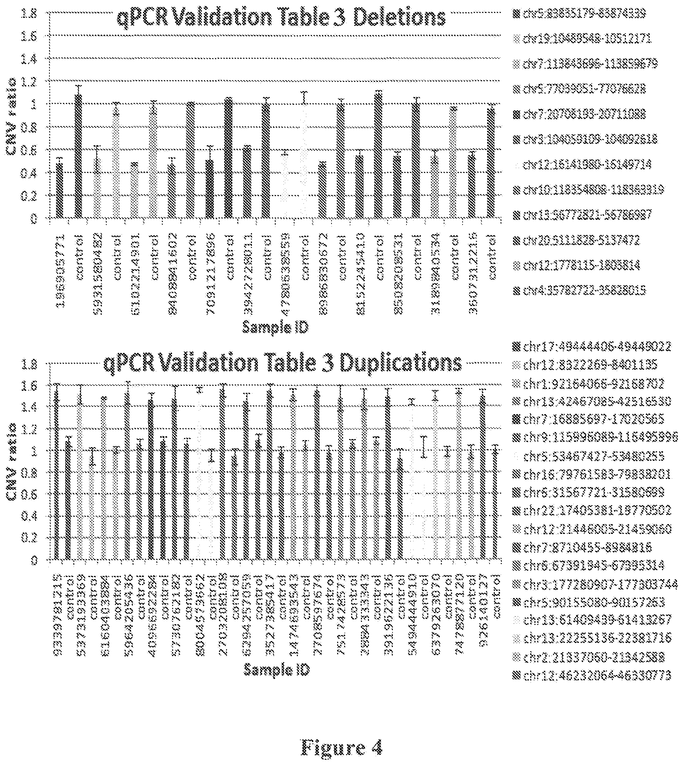

34 putative CNVR loci (15 deletions and 19 duplications) were identified that were exclusively present in at least three EA cases ($P \leq 0.05$). However, three of the deletions proved to be false positives during the validation process with quantitative PCR (qPCR), a method commonly used for independent validation of CNVs (Table 3 and Figure 4). All of the experimental validations were conducted in a blinded manner by an independent investigator group to minimize any bias, which adds another level of confidence to the experimental validation results. Issues related to CNV recurrence, accuracy, locus dependence and difficulties related to GC content were alleviated by running qPCR in quadruplicate, observing low standard deviation across runs, assaying control samples for the same genomic region and assaying the same samples in different genomic regions.

**Table 3:** CNVs at the SNP level (qPCR validated but not filtered for presence in the public domain) exclusive to childhood obesity in the European American cohort and replication attempt in African Americans. Those CNVs that replicated exclusively in African American cases are highlighted in bold. Co-ordinates are derived from UCSC Build hg 18. The "BP from nearest gene" column lists zero if the CNV encompasses the gene listed or a value if the CNV is nearby the gene listed. The individual CNV calls are assigned as a SNP-based statistic. Therefore, contiguous SNPs showing case non-zero values and control zero values form a region of CNV which are the CNVRs listed. The SNP tested is the central SNP in the CNVR region to ensure exact overlap in replication.

| CNV | European Americans Cases impacted exclusively | African Americans SNP tested | Cases impacted | Controls impacted | BP from nearest gene | Nearest gene |
|---|---|---|---|---|---|---|
| DELETIONS | | | | | | |
| **chr5:83835179-83874339** | **5** | **rs10051401** | **1** | **0** | **118812** | **EDIL3** |
| **chr19:10489548-10512171** | **3** | **rs11670254** | **5** | **0** | **0** | **S1PR5 (EDG8)** |
| **chr7:113843696-113859679** | **3** | **rs12705964** | **3** | **0** | **0** | **FOXP2** |
| **chr5:77039051-77076628** | **3** | **rs384109** | **2** | **0** | **0** | **TBCA** |
| **chr7:20708193-20711088** | **3** | **rs12700232** | **1** | **0** | **0** | **ABCB5** |
| **chr3:104059109-104092618** | 3 | **rs1144781** | **1** | **0** | **377734** | **ZPLD1** |
| chr12:16141980-16149714 | 3 | rs1376332 | 0 | 0 | 60398 | DERA |
| chr10:118354808-118363319 | 3 | rs7905887 | 0 | 1 | 0 | PNLIPRP1 |
| chr13:56772821-56786987 | 3 | rs7981921 | 74 | 94 | 130468 | FLJ40296 |
| chr20:5111828-5137472 | 3 | rs17785402 | 0 | 0 | 0 | BC015432, CDS2 |
| chr12:1778115-1803814 | 3 | rs11611260 | 2 | 1 | 0 | CACNA2D4, LRTM2 |
| chr4:35782722-35828015 | 3 | rs4833119 | 1 | 1 | 0 | CENTD1 |
| | | | | | | |
| DUPLICATIONS | | | | | | |
| **chr17:49444406-49449022** | **3** | **rs17730346** | **2** | **0** | **186834** | **KIF2B** |
| **chr12:8322269-8401135** | **3** | **rs10770444** | **1** | **0** | **0** | **BC073935, CR611653** |
| **chr1:92164066-92168702** | **3** | **rs11165816** | **1** | **0** | **19018** | **BRDT** |
| **chr13:42467085-42516530** | **3** | **rs2057529** | **1** | **0** | **0** | **DNAJC15** |

| DUPLICATIONS | | | | | | |
|---|---|---|---|---|---|---|
| **chr7:16885697-17020565** | **3** | **rs847403** | **1** | **0** | **0** | **AGR3** |
| chr9:115996089-116495996 | 3 | rs942520 | 1 | 0 | 0 | **DFNB31, COL27A1, AKNA, ATP6V1G1, ORM1, ORM2, c9orf91** |
| **chr5:53467427-53480255** | **3** | **rs16882296** | **1** | **0** | **0** | **ARL15** |
| chr16:79761583-79838201 | 4 | rs12933806 | 0 | 0 | 0 | PKD1L2, BCMO1 |
| chr6:31567721-31580699 | 3 | rs3828903 | 0 | 0 | 0 | MICB |
| chr22:17405381-19770502 | 3 | rs2238790 | 3 | 4 | 0 | COMT+ |
| chr12:21446005-21459060 | 3 | rs7953750 | 0 | 0 | 6367 | SLCO1A2 |
| chr7:8710455-8984816 | 3 | rs1859416 | 0 | 0 | 0 | NXPH1 |
| chr6:67391945-67395314 | 3 | rs183895 | 0 | 2 | 918106 | EGFL11 |
| chr3:177280907-177303744 | 3 | rs9875122 | 0 | 0 | 274785 | NAALADL2 |
| chr5:90155080-90157263 | 3 | rs7717221 | 0 | 0 | 0 | GPR98 |
| chr13:61409439-61413267 | 3 | rs2183953 | 0 | 0 | 509359 | PCDH20 |
| chr13:22255136-22381716 | 3 | rs9510354 | 0 | 0 | 0 | BC048997, AK091626 |
| chr2:21337060-21342588 | 3 | rs870638 | 0 | 0 | 216610 | APOB |
| chr12:46232064-46330773 | 3 | rs739856 | 0 | 0 | 12568 | RPAP3 |

In the replication attempt, 13 of the 31 validated CNVR loci (41.9%) were also found to be exclusively present in one or more AA cases (6 deletions and 7 duplications) (Table 3). Only 17 of the CNVR loci were unique to the instant cohort (i.e., not reported in controls by the Database of Genomic Variants), of which 8 (47.1%) also replicated exclusively in AA cases (6 deletions and 2 duplications) (Table 4). The use of a different racial group for replication purposes represents a higher bar than making similar attempts in the same ethnicity. For example, when the common variant in *TCF7L2* (MIM 602228) associated with type 2 diabetes was also associated with the disease in Africans with a similar magnitude (Helgason et al. (2007) Nat. Genet., 39:218-225), it was considered much more established as having a role in the disease; in addition, the recent asthma GWAS finding was also observed in African Americans which gave us a much higher level of certainty of the observation (Sleiman et al. (2010) New Eng. J. Med., 362:36-44). Since many variants have been shown to have different frequencies in different ethnic groups, observation of the same CNV as exclusive in EA and AA cohorts separately for the same phenotype minimizes the potential for confounding effects of population stratification. Figure 3 visualizes representative loci most frequently impacted by deletions or duplications using the UCSC Genome Browser with Build hg18 (March 2006; genome.ucsc.edu/ cgi-bin/hgGateway) of the human genome. It was also evaluated if any of the key CNVs co-segregated with obesity in families, where data was available, and were able to establish that at least four of the CNVs were inherited from an obese parent and at least four were *de novo* (Table 5).

**Table 4:** CNVs at the SNP level (both not present in the public domain and qPCR validated) exclusive to childhood obesity in the European American cohort and replication attempt in African Americans. Those CNVs that replicated exclusively in African American cases are highlighted in bold. Co-ordinates are derived from UCSC Build hg18. The "BP from nearest gene" column lists zero if the CNV encompasses the gene listed or a value if the CNV is nearby the gene listed. These loci showed CN=1 for deletion loci and CN=3 for duplication loci across contributing samples. No other states such as CN=0 or CN=4 were observed at these loci. The individual CNV calls are assigned as a SNP-based statistic. Therefore, contiguous SNPs showing case non-zero values and control zero values form a region of CNV which are the CNVRs listed. The SNP tested is the central SNP in the CNVR region to ensure exact overlap in replication.

| | European Americans | African Americans | | | | |
|---|---|---|---|---|---|---|
| CNV | Cases impacted exclusively | SNP tested | Cases impacted | Controls impacted | BP from nearest gene | Nearest gene |
| DELETIONS | | | | | | |
| **chr5:83835179-83874339** | **5** | **rs10051401** | **1** | **0** | **118812** | **EDIL3** |
| **chr19:10489548-10512171** | **3** | **rs11670254** | **5** | **0** | **0** | **S1PR5 (EDG8)** |
| **chr7:113843696-113859679** | **3** | **rs12705964** | **3** | **0** | **0** | **FOXP2** |
| **chr5:77039051-77076628** | **3** | **rs384109** | **2** | **0** | **0** | **TBCA** |
| **chr7:20708193-20711088** | **3** | **rs12700232** | **1** | **0** | **0** | **ABCB5** |
| **chr3:104059109-104092618** | **3** | **rs1144781** | **1** | **0** | **377734** | **ZPLD1** |
| chr12:16141980-16149714 | 3 | rs1376332 | 0 | 0 | 60398 | DERA |
| chr10:118354808-118363319 | 3 | rs7905887 | 0 | 1 | 0 | PNLIPRP1 |
| chr20:5111828-5137472 | 3 | rs17785402 | 0 | 0 | 0 | BC015432, CDS2 |
| chr4:35782722-35828015 | 3 | rs4833119 | 1 | 1 | 0 | CENTD1 |
| | | | | | | |
| DUPLICATIONS | | | | | | |
| **chr17:49444406-49449022** | **3** | **rs17730346** | **2** | **0** | **186834** | **KIF2B** |
| **chr5:53467427-53480255** | **3** | **rs16882296** | **1** | **0** | **0** | **ARL15** |
| chr12:21446005-21459060 | 3 | rs7953750 | 0 | 0 | 6367 | SLCO1A2 |
| chr6:67391945-67395314 | 3 | rs183895 | 0 | 2 | 918106 | EGFL11 |
| chr5:90155080-90157263 | 3 | rs7717221 | 0 | 0 | 0 | GPR98 |
| chr13:61409439-61413267 | 3 | rs2183953 | 0 | 0 | 509359 | PCDH20 |

(continued)

| DUPLICATIONS | | | |
|---|---|---|---|
| chr2:21337060-21342588 | 3 | rs870638 | 0 | 0 | 216610 | APOB |

**Table 5:** Inheritance of CNVs observed, based on available family data.

| CNVR | Inheritance |
|---|---|
| chr1:162647580-162655215 | *de novo* |
| chr5:83835179-83874339 | From obese father |
| chr19:10489548-10512171 | Not from mother |
| chr19:10489548-10512171 | Not from mother |
| chr7:20708193-20711088 | *de novo* |
| chr12:16141980-16149714 | From obese father |
| chr10:118354808-118363319 | From mother |
| chr13:56772821-56786987 | Not from mother |
| chr4:35782722-35828015 | Not from mother |
| chr17:49444406-49449022 | From obese mother |
| chr17:49444406-49449022 | Not from mother |
| chr17:49444406-49449022 | *de novo* |
| chr13:42467085-42516530 | From obese mother |
| chr7:16885697-17020565 | From mother |
| chr5:53467427-53480255 | Not from mother |
| chr16:79761583-79838201 | Not from mother |
| chr16:79761583-79838201 | From father |
| chr6:31567721-31580699 | *de novo* |
| chr3:177280907-177303744 | Not from mother |
| chr13:22255136-22381716 | From mother |
| chr13:22255136-22381716 | Not from mother |
| chr2:21337060-21342588 | Not from mother |
| chr12:46232064-46330773 | Not from mother |

In order to further establish the significance of these findings, the other tail of the distribution (i.e. exclusive CNVRs among the controls) was examined. Firstly, while exclusivity to three obese subjects constituted a nominally significant observation among EA cases, ten EA controls were required to reach the same significance threshold due to the abundance of controls in the study. Analyzed in this way, no exclusive deletions were observed and only four exclusive duplications in the EA controls were observed. However, none of these duplications replicated exclusively in the AA controls. As such, in contrast to these control observations, a highly significant over abundance of exclusive CNVRs was seen in the EA cases that go on to replicate exclusively in the AA cases, revealing that there is less than a 12% false positive discovery rate.

Large rare deletions present in <1% of individuals and >500 kb in size as set previously (Bochukova et al. (2010) Nature 463:666-670) were evaluated and an excess of large rare deletions genome-wide was not observed (Table 6). This is not unexpected given the previous report only found significance when including developmental delay subjects but not when severe early-onset obesity was evaluated alone.

**Table 6:** Comparison of large rare CNV load genome-wide.

| Sample Cohort | CNV Type | Case Frequency | Control Frequency | *P* value |
|---|---|---|---|---|
| **European Americans** | Deletions | 0.0222 | 0.0224 | 1 |
| | Duplications | 0.0611 | 0.0588 | 0.817 |
| | Deletions and Duplications | 0.0824 | 0.0760 | 0.541 |
| **African Americans** | Deletions | 0.0128 | 0.0184 | 0.245 |
| | Duplications | 0.0345 | 0.0406 | 0.393 |
| | Deletions and Duplications | 0.0541 | 0.0584 | 0.638 |

The loci harboring exclusive deletions in cases of both ethnicities consisted of four genes directly impacted, namely *S1PR5* (endothelial differentiation, sphingolipid; MIM605146), *FOXP2* (forkhead box P2; MIM 605317), *TBCA* (tubulin-specific chaperone a; MIM 610058) and *ABCB5* (ATP-binding cassette, sub-family B, member 5; MIM 611785) while two deletions were close to *EDIL3* (EGF-like repeats- and discoidin I-like domains-containing protein 3; MIM 606018) and *ZPLD1* (zona pellucida-like domain containing 1), respectively.

The loci harboring exclusive duplications in cases from both ethnicities consisted of four genes directly impacted, namely *BC073935-CR611653, DNAJC15* (DnaJ homolog, subfamily C, member 15), *AGR3* (breast cancer membrane protein 11 precursor) and *ARL15* (ADP-ribosylation factor-like 15) plus a gene cluster at 9q32. In addition, two duplications were close to *KIF2B* (kinesin family member 2B) and *BRDT* (testis-specific bromodomain protein).

Pairwise IBD, both global and local, yielded low values close to zero between the five cases with the EDIL3 deletion; although exact CNV breakpoint sharing between cases was not a necessary parameter of the analysis, it does indicate that a specific copy number polymorphism is observed. The two loci harboring exclusive duplications in cases from both ethnicities consisted of one gene directly impacted, namely ARL15 (ADP-ribosylation factor-like 15) and one closest to KIF2B (kinesin family member 2B).

The majority of genes harboring at the loci uncovered in this study have not been implicated in obesity previously. However, the most notable finding is with *ARL15,* which was recently uncovered in a GWA study of adiponectin levels, with the same risk allele also being associated with a higher risk of coronary heart disease and type 2 diabetes (Richards et al. (2009) PLoS Genet., 5:e1000768).

In addition, although members of the human Forkhead-box (FOX) gene family have been strongly implicated in metabolic traits (Katoh et al. (2004) Int. J. Oncol., 25:1495-500), mutations in FOXP2 are best known for causing developmental speech and language disorders in humans (Vernes et al. (2008) N. Engl. J. Med., 359:2337-45). Although autism was recorded for one EA case and ADHD for one AA case (the only one of the replicated loci with reported co-morbidities), none of the children impacted by FOXP2 CNVs had evidence of classical syndromes. The connection between the development of human language and speech and childhood obesity is not clear but one could hypothesize that problems with speech could lead to greater likelihood of social isolation and thus less activity. Indeed, this theory is supported by the fact that there are clear speech and language characteristics in children with Prader-Willi syndrome, who generally present with morbid obesity (Akefeldt et al. (1997) J. Intellect Disabil. Res., 41:302-11; Kleppe et al. (1990) J. Speech Hear. Disord., 55:300-9).

The EA cohort was used for discovery due to the much larger control group available in this ethnicity to observe and establish exclusivity most comprehensively in the discovery setting. However, in order to explore the possibility of additional exclusive loci, the AA cohort was also analyzed as the discovery cohort and attempted to replicate in the EA cohort (Table 7). Although exclusivity to five AA cases was required to achieve nominal significance in the discovery stage in this setting due to fewer lean controls in this cohort (Table 8), evidence for a deletion at the EPHA6-UNQ6114 locus (MIM 600066; e.g., www.ncbi.nlm.nih.gov/omim/ 600066) was observed, that was exclusive to AA cases.

**Table 7:** African American cohort as discovery cohort and European Americans as replication. Those CNVs that replicated exclusively in European American cases are highlighted in bold. Note that a minimum of 5 CNVs exclusive in African American Cohort were required to reach nominal significance (P ≤ 0.05) in discovery, mainly due to the lower number of lean African Americans in the cohort relative to lean European Americans.

| CNV | African Americans | | European Americans | | BP from nearest gene | Nearest gene |
|---|---|---|---|---|---|---|
| | Cases impacted exclusively | SNP tested | Cases impacted | Controls impacted | | |
| <u>DELETIONS</u> | | | | | | |
| **chr3:97523082-97528441** | **5** | **rs9843398** | **1** | 0 | **487674** | ***EPHA6,UNQ 6114*** |
| **chr19:10477303-10512171** | **5** | **rs12460981** | **3** | **0** | **0** | ***S1PR5*** |
| chr3:105183599-105186764 | 6 | rs2166826 | 0 | 3 | 0 | *B070396* |
| chr5:84245129-84399973 | 6 | rs1154964 | 2 | 3 | 528762 | *EDIL3* |
| chr8:115226631-115278440 | 5 | rs1515685 | 0 | 0 | 708213 | *CSMD3* |
| chr6:120618851-120652337 | 5 | rs9320745 | 0 | 2 | 764685 | *AK097101* |
| | | | | | | |
| <u>DUPLICATIONS</u> | | | | | | |
| chr3:120225538-120284544 | 5 | rs7638463 | 5 | 12 | 0 | *IGSF11* |

**Table 8:** Discovery significance justification for frequency thresholds.

| EA Cases Harboring | EA Cases Not Harboring | EA Controls Harboring | EA Controls Not Harboring | P value (Fisher's Exact Test Two Tailed) |
|---|---|---|---|---|
| 0 | 1080 | 0 | 2500 | 1 |
| 1 | 1079 | 0 | 2500 | 0.301676 |
| 2 | 1078 | 0 | 2500 | 0.09095 |
| 3 | 1077 | 0 | 2500 | 0.027402 |
| 4 | 1076 | 0 | 2500 | 0.00825 |
| 5 | 1075 | 0 | 2500 | 0.002483 |
| 6 | 1074 | 0 | 2500 | 0.000746 |
| 7 | 1073 | 0 | 2500 | 0.000224 |
| 8 | 1072 | 0 | 2500 | $6.74 \times 10^{-5}$ |
| 9 | 1071 | 0 | 2500 | $2.02 \times 10^{-5}$ |
| 10 | 1070 | 0 | 2500 | $6.06 \times 10^{-6}$ |

| AA Cases Harboring | AA Cases Not Harboring | AA Controls Harboring | AA Controls Not Harboring | P value (Fisher's Exact Test Two Tailed) |
|---|---|---|---|---|
| 0 | 1479 | 0 | 1575 | 1 |
| 1 | 1478 | 0 | 1575 | 0.484283 |
| 2 | 1477 | 0 | 1575 | 0.234448 |
| 3 | 1476 | 0 | 1575 | 0.11346 |
| 4 | 1475 | 0 | 1575 | 0.054889 |
| 5 | 1474 | 0 | 1575 | 0.026545 |
| 6 | 1473 | 0 | 1575 | 0.012833 |
| 7 | 1472 | 0 | 1575 | 0.006202 |
| 8 | 1471 | 0 | 1575 | 0.002996 |
| 9 | 1470 | 0 | 1575 | 0.001447 |
| 10 | 1469 | 0 | 1575 | 0.000698 |

To date, there has been a notable paucity of GWA studies in childhood obesity, with studies exclusively uncovering loci in the adult setting (Frayling et al. (2007) Science 316:889-94; Grant et al. (2008) PLoS ONE 3:e1746; Hinney et al. (2007) PLoS ONE 2:e1361; Dina et al. (2007) Nat. Genet., 39:724-6; Scuteri et al. (2007) PLoS Genet., 3:e115; Loos et al. (2008) Nat. Genet., 40:768-75; Thorleifsson et al. (2009) Nat. Genet., 41:18-24; Willer et al. (2009) Nat. Genet., 41:25-34), and no study to date has reported CNVs that are significantly associated with the non-syndromic trait as opposed to the syndromic form (Bochukova et al. (2010) Nature 463:666-70; Walters et al. (2010) Nature 463:671-5). As such, the instant study represents the first large-scale, unbiased genome-wide scan of CNVs in pediatric obesity. The loci uncovered in this present study are for the first time exclusively observed in childhood obesity and replicated in an independent case control data set from a different ethnicity. The results are given extra credibility as the well-established FTO (Frayling et al. (2007) Science 316:889-94; Grant et al. (2008) PLoS ONE 3:e1746; Hinney et al. (2007) PLoS ONE 2:e1361; Dina et al. (2007) Nat. Genet., 39:724-6; Scuteri et al. (2007) PLoS Genet., 3:e115) locus is very strongly associated with both BMI and obesity in the EA cohort (Grant et al. (2008) PLoS One, 3:e1746; Zhao et al. (2009) Examination of all type 2 diabetes GWAS loci reveals HHEX-IDE as a locus influencing pediatric BMI. Diabetes). Interestingly, no association was observed with any CNVs previously reported in extreme syndromic pediatric obesity with developmental delay (Bochukova et al. (2010) Nature 463:666-70). In addition, the previously reported common CNV flanking NEGR1 (MIM 613173) or encompassing SH2B1 (MIM 608937) (Willer et al. (2009) Nat. Genet., 41:25-34) were not further studied.

Taken together, the instant unbiased approach to assess the entire genome has revealed novel genes impacted by CNVs that are exclusive to cases in two different ethnicities and have not previously been directly implicated in the context of obesity and await further characterization. Further functional studies may be performed to more fully characterize the function of the genes at these loci in relation to childhood obesity.

**Claims**

1. An in vitro method for diagnosing an increased risk for non-syndromic obesity in a child subject, said method comprising determining the copy number of at least one gene in a biological sample from said subject,

wherein said gene is selected from the group consisting of EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3, and EPHA6,

wherein the presence of a variation in the copy number of at least one of said genes is indicative of an increased risk for obesity in a Caucasian child subject; and.

wherein the presence of a variation in the copy number of at least one of EDIL3, S 1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91, ARL15, and EPHA6 is indicative of an increased risk for obesity in a child subject of African ancestry.

2. The method of claim 1, wherein said gene is selected from the group consisting of EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91, and ARL15.

3. The method of claim 1, wherein said gene is selected from the group consisting of EDIL3, S1PR5, FOXP2, KIF2B, ARL15, and DNAJC15.

4. The method of claim 1, wherein said variation in copy number is a deletion.

5. The method of claim 1, wherein said variation in copy number is a duplication.

6. An in vitro method for identifying agents which are therapeutic for the treatment of obesity, said method comprising:

a) providing cells expressing at least one copy number variant, wherein said copy number variant is of at least one gene selected from the group consisting of EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3, and EPHA6;

b) contacting the cells of step a) with a test agent; and

c) determining whether said agent modulates an activity of said gene, wherein the modulation of the activity of the copy number variant towards wild-type activity is indicative of a therapeutic agent.

7. The method of claim 6, wherein said cells of step a) are in a non-human animal.

8. An array of array elements on a solid support, wherein said array elements consist of at least six probes, and wherein each of said probes specifically hybridizes to a unique gene selected from the group consisting of EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3, and EPHA6.

9. The array of claim 8, wherein said array elements consist of at least one probe to each of EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91, and ARL15.

10. The array of claim 9, wherein said array elements consist of at least one probe to each of EDIL3, S1PR5, FOXP2, KIF2B, ARL15, and DNAJC15.

11. The array of claim 8, wherein said array elements consist of at least one probe to each of the genes listed.

**Patentansprüche**

1. In-Vitro-Verfahren zur Diagnose eines erhöhten Risikos für nicht-syndromale Adipositas (Fettleibigkeit) in einem Kind, wobei das Verfahren die Bestimmung der Kopienzahl von zumindest einem Gen in einer biologischen Probe aus dem Kind umfasst,
   wobei das Gen aus der Gruppe ausgewählt ist, die aus EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3 und EPHA6 besteht,
   wobei die Anwesenheit einer Kopienzahlvariation von zumindest einem der Gene hinweisend auf ein erhöhtes Risiko für Adipositas in einem Kind kaukasischer Abstammung ist; und.
   wobei die Anwesenheit einer Kopienzahlvariation von zumindest einem aus EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91, ARL15 und EPHA6 hinweisend auf ein erhöhtes Risiko für Adipositas in einem Kind afrikanischer Abstammung ist.

2. Verfahren nach Anspruch 1, wobei das Gen aus der Gruppe ausgewählt ist, die aus EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91 und ARL15 besteht.

3. Verfahren nach Anspruch 1, wobei das Gen aus der Gruppe ausgewählt ist, die aus EDIL3, S1PR5, FOXP2, KIF2B, ARL15 und DNAJC15 besteht.

4. Verfahren nach Anspruch 1, wobei die Kopienzahlvariation eine Deletion ist.

5. Verfahren nach Anspruch 1, wobei die Kopienzahlvariation eine Duplikation ist.

6. In-Vitro-Verfahren zum Identifizieren von Mitteln, die für die Behandlung von Adipositas therapeutisch wirksam sind, wobei das Verfahren umfasst:

   a) Bereitstellen von Zellen, die zumindest eine Kopienzahlvariante exprimieren, wobei die Kopienzahlvariante zumindest ein Gen ist, das aus der Gruppe ausgewählt ist, die aus EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3 und EPHA6 besteht;
   b) In-Kontakt-Bringen der Zellen aus Schritt a) mit einem Testmittel; und
   c) Bestimmen, ob das Mittel eine Aktivität des Gens verändert, wobei das Verändern der Aktivität in der Kopienzahlvariante gegenüber der Wildtypaktivität hinweisend auf ein therapeutisches Mittel ist.

7. Verfahren nach Anspruch 6, wobei die Zellen aus Schritt a) in einem nicht-humanen Tier vorkommen.

8. Array mit Arrayelementen auf einem festen Träger, wobei die Arrayelemente aus zumindest sechs Sonden bestehen, und wobei jede der Sonden spezifisch mit einem einzigen Gen hybridisiert, das aus der Gruppe ausgewählt ist, die aus EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3 und EPHA6 besteht.

9. Array nach Anspruch 8, wobei die Arrayelemente aus zumindest einer Sonde für jedes Gen aus EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91 und ARL15 bestehen.

10. Array nach Anspruch 9, wobei die Arrayelemente aus zumindest einer Sonde für jedes Gen aus EDIL3, S1PR5, FOXP2, KIF2B, ARL15 und DNAJC15 bestehen.

11. Array nach Anspruch 8, wobei die Arrayelemente aus zumindest einer Sonde für jedes der aufgezählten Gene bestehen.

## Revendications

1. Procédé *in vitro* de diagnostic d'un risque accu d'obésité non syndromique chez un patient enfant, ledit procédé comprenant la détermination du nombre de copies d'au moins un gène dans un échantillon biologique provenant dudit patient,

   dans lequel ledit gène est choisi dans le groupe constitué par EDIL3, S1 PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3 et EPHA6,

   dans lequel la présence d'une variation du nombre de copies d'au moins un desdits gènes indique un risque accru d'obésité chez un patient enfant de type caucasien ; et

   dans lequel la présence d'une variation du nombre de copies d'au moins l'un de EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91, ARL15 et EPHA6 indique un risque accru d'obésité chez un patient enfant d'origine africaine.

2. Procédé selon la revendication 1, dans lequel ledit gène est choisi dans le groupe constitué par EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91 et ARL15.

3. Procédé selon la revendication 1, dans lequel ledit gène est choisi dans le groupe constitué par EDIL3, S1PR5, FOXP2, KIF2B, ARL15 et DNAJC15.

4. Procédé selon la revendication 1, dans lequel ladite variation du nombre de copies est une délétion.

5. Procédé selon la revendication 1, dans lequel ladite variation du nombre de copies est une duplication.

6. Procédé *in vitro* d'identification d'agents thérapeutiques pour le traitement de l'obésité, ledit procédé comprenant les étapes consistant à :

   a) fournir des cellules exprimant au moins un variant du nombre de copies, dans lequel ledit variant du nombre de copies est d'au moins un gène choisi dans le groupe constitué par EDIL3, S1PR5, FOXP2, TBCA, ABCB5, ZPLD1, DERA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3 et EPHA6 ;

   b) mettre en contact les cellules de l'étape a) avec un agent d'essai ; et

   c) déterminer si ledit agent module une activité dudit gène, dans lequel la modulation de l'activité du variant du nombre de copies vis-à-vis de l'activité de type sauvage indique un agent thérapeutique.

7. Procédé selon la revendication 6, dans lequel lesdites cellules de l'étape a) sont dans un animal non humain.

8. Réseau d'éléments de réseau sur un support solide, dans lequel les éléments de réseau sont constitués d'au moins six sondes, et dans lequel chacune desdites sondes s'hybride spécifiquement à un gène unique choisi dans le groupe constitué par EDIL3, S1PR5, FOXP2, TBCA, ABCB5, ZPLD1, DEKA, PNLIPRP1, PRR20A, BC015432, CDS2, CACNA2D4, LRTM2, CENTD1, KIF2B, BC073935, CR611653, BRDT, DNAJC15, AGR3, DFNB31, COL27A1, AKNA, ATP6V1G1, ORM1, ORM2, C9orf91, ARL15, PKD1L2, BCMO1, MICB, COMT, SLCO1A2, NXPH1, EGFL11, NAALADL2, GPR98, PCDH20, BC048997, AK091626, APOB, RPAP3 et EPHA6.

9. Réseau selon la revendication 8, dans lequel lesdits éléments de réseau sont constitués d'au moins une sonde à chacun de EDIL3, S1PR5, FOXP2, TBCA, ABCB5, ZPLD1, KIF2B, BC073935, CR611653, BRDT, DNAJC15, AGR3, DFNB31, COL27A1, AKNA, ATP6V1G1, ORM1, ORM2, C9orf91 et ARL15.

10. Réseau selon la revendication 9, dans lequel lesdits éléments de réseau sont constitués d'au moins une sonde à chacun de EDIL3, S1PR5, FOXP2, KIF2B, ARL15 et DNAJC15.

**11.** Réseau selon la revendication 8, dans lequel lesdits éléments de réseau sont constitués d'au moins une sonde à chacun des gènes énumérés.

Figure 1

**C** **Obesity Cases African American**

**D** **Lean Controls African Americans**

**Figure 1**

Figure 2

chr5 (q14.3)

Window Position
chr5

Human Mar. 2006   chr5:83,561,052-84,148,466 (587,415 bp)

83850000 | 83700000 | 83750000 | 83800000 | 83850000 | 83900000 | 83950000 | 84000000 | 84050000 | 84100000 |

V1V3Q610intSNPs

Illumina Infinium Human V1V3Q610 Intersection SNPs

EA CHOP Obesity Homozygous Deletion
EA CHOP Obesity Control Homozygous Deletion
AA CHOP Obesity Homozygous Deletion
AA CHOP Obesity Controls Homozygous Deletion
EA CHOP Obesity Deletion

1848100601_A.1.v3
1800835416_A.1.v3
1862260783_A.1.v3
4290041067_R02C02.2
4523247141_R02C02.3
4290041604_R02C01.2

EA CHOP Obesity Control Deletion

1894405279_A.1.v3
1962666301_A.2.v3
1964366213_A.2.v3
4243114142_R01C01.2

AA CHOP Obesity Deletion

1562065297_A
1671113064_A
4278098040_R01C01

AA CHOP Obesity Controls Deletion

1873031334_A
1780848093_A
1873031334_A

RefSeq Genes

EDIL3

Database of Genomic Variants: Structural Variation (CNV, Inversion, Indel)

36432
53582

36433
22791
23150
23775
37819

Duplications of >1000 Bases of Non-RepeatMasked Sequence
chr12:39383398

**Figure 3A**

Figure 3B

Figure 3C

Figure 3D

Figure 3E

Figure 3F

Figure 4

| Normalize θ to B-Allele Frequency (BAF) and R to Log R Ratio (LRR) |

| Quality Control the sample set | |
|---|---|
| Metric | Excluded |
| Call_Rate > 98% | 69 |
| SD_LRR < 0.3 | 208 |
| -0.1<GCWF<0.1 | 0 |
| Count CNV < 100 | 115 |
| Ethnicity | 0 |

| BAF Values | LRR Values |
|---|---|
| Population frequency of B alleles | SNP Genome coordinates |

**Sample Input**

Trained HMM Model File

**Definition Files**

PennCNV

CNV Calls

SNP based Population CNV Statistics for deletions and duplications (8,672 del and 6,763 dup)

CNVR Definition Based on similar significance    -log p value ± 1

272 deletion 174 duplication CNVRs nominally significant (21 del and 21 dup exclusive)

Review
Telomere, centromere, CNV peninsula, GC content, SNP coverage, CNVR sample bias, Database for Genomic Variants, qPCR, exclusive

12 deletion and 19 duplication CNVRs

Replicate in Africans

6 deletion and 7 duplication CNVRs

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683195 A
- US 4800195 A
- US 4965188 A

### Non-patent literature cited in the description

- **TROIANO et al.** *Pediatrics,* 1998, vol. 101, 497-504
- **REAVEN, G.M.** *Diabetes,* 1988, vol. 37, 1595-607
- **DEFRONZO et al.** *Diabetes Care,* 1991, vol. 14, 173-94
- Ann. Intern. Med. National Institutes of Health Consensus Development Conference Statement, 1985, vol. 103, 147-51
- **NICKLAS et al.** *J. Am. Coll. Nutr.,* 2001, vol. 20, 599-608
- **WHITAKER et al.** *N. Engl. J. Med.,* 1997, vol. 337, 869-73
- **PARSONS et al.** *Int. J. Obes. Relat. Metab. Disord.,* 1999, vol. 23 (8), 1-107
- **MUST, A.** *Nutr. Rev.,* 2003, vol. 61, 139-42
- **FRIEDMAN, J.M.** *Nat. Med.,* 2004, vol. 10, 563-9
- **LYON et al.** *Am. J. Clin. Nutr.,* 2005, vol. 82, 215S-217S
- **KNOWLER et al.** *Diabetes Metab. Rev.,* 1990, vol. 6, 1-27
- **ZIMMET et al.** *Diabetes Metab. Rev.,* 1990, vol. 6, 91-124
- **STUNKARD et al.** *JAMA,* 1986, vol. 256, 51-4
- **BORJESON et al.** *Acta Paediatr. Scand.,* 1976, vol. 65, 279-87
- **HEBEBRAND et al.** *Obes. Rev.,* 2003, vol. 4, 139-46
- **FAROOQI et al.** *Int. J. Obes. (Lond),* 2005, vol. 29, 1149-52
- **BELL et al.** *Nat. Rev. Genet.,* 2005, vol. 6, 221-34
- **SCHOUSBOE et al.** *Twin Res.,* 2003, vol. 6, 409-21
- **HERBERT et al.** *Science,* 2006, vol. 312, 279-83
- **LOOS et al.** *Science,* 2007, vol. 315, 187
- **DINA et al.** *Science,* 2007, vol. 315, 187
- **ROSSKOPF et al.** *Science,* 2007, vol. 315, 187
- **LYON et al.** *PLoS Genet.,* 2007, vol. 3, e61
- **HOTTA et al.** *J. Hum. Genet.,* 2008, vol. 53, 857-62
- **FRAYLING et al.** *Science,* 2007, vol. 316, 889-94
- **GRANT et al.** *PLoS ONE,* 2008, vol. 3, e1746
- **HINNEY et al.** *PLoS ONE,* 2007, vol. 2, e1361
- **DINA et al.** *Nat. Genet.,* 2007, vol. 39, 724-6
- **SCUTERI et al.** *PLoS Genet.,* 2007, vol. 3, e115
- **LOOS et al.** *Nat. Genet.,* 2008, vol. 40, 768-75
- **THORLEIFSSON et al.** *Nat. Genet.,* 2009, vol. 41, 18-24
- **WILLER et al.** *Nat. Genet.,* 2009, vol. 41, 25-34
- **BOCHUKOVA et al.** *Nature,* 2010, vol. 463, 666-70
- **SAMBROOK et al.** Molecular Cloning: A Laboratory Manual. Cold Spring Harbor Laboratory Press, 1989
- Remington's Pharmaceutical Sciences. **E.W. MARTIN.** Remington's Pharmaceutical Sciences. Mack Publishing Co, 1990
- Remington: The Science and Practice of Pharmacy. Lippincott Williams & Wilkins
- **WALTERS et al.** *Nature,* 2010, vol. 463, 671-5
- **DISKIN et al.** *Nucleic Acids Res.,* 2008, vol. 36, e126
- **DENNIS et al.** *Genome Biol.,* 2003, vol. 4, 3
- **HUANG DA et al.** *Genome Biol.,* 2007, vol. 8, R183
- **WANG et al.** *Genome Res.,* 2007, vol. 17, 1665-74
- **HELGASON et al.** *Nat. Genet.,* 2007, vol. 39, 218-225
- **SLEIMAN et al.** *New Eng. J. Med.,* 2010, vol. 362, 36-44
- **BOCHUKOVA et al.** *Nature,* 2010, vol. 463, 666-670

- **RICHARDS et al.** *PLoS Genet.,* 2009, vol. 5, e1000768
- **KATOH et al.** *Int. J. Oncol.,* 2004, vol. 25, 1495-500
- **VERNES et al.** *N. Engl. J. Med.,* 2008, vol. 359, 2337-45
- **AKEFELDT et al.** *J. Intellect Disabil. Res.,* 1997, vol. 41, 302-11

- **KLEPPE et al.** *J. Speech Hear. Disord.,* 1990, vol. 55, 300-9
- **GRANT et al.** *PLoS One,* 2008, vol. 3, e1746
- **ZHAO et al.** Examination of all type 2 diabetes GWAS loci reveals HHEX-IDE as a locus influencing pediatric BMI. *Diabetes,* 2009